# EUROPEAN PATENT APPLICATION

(11) **EP 4 067 474 A1**
(43) Date of publication of application: **05.10.2022**
(21) Application number: 20894154.2
(22) Date of filing: 27.11.2020
(51) Int. Cl.: C12M 3/02, C12N 5/02, B01J 20/26, C12P 21/02

(54) **CELL CULTURE METHOD, ANTIBODY PRODUCTION METHOD, ORGANIC ACID REMOVAL METHOD, AND ANTIBODY**

(30) Priority: 29.11.2019 JP 2019217060
(71) Applicant: FUJIFILM Corporation, Tokyo 106-8620 (JP)
(72) Inventor: SHIMONO, Katsuhiro, Ashigarakami-gun, Kanagawa 258-8577 (JP); HIRAI, Yoshinobu, Ashigarakami-gun, Kanagawa 258-8577 (JP); ISHIHARA, Tsukasa, Ashigarakami-gun, Kanagawa 258-8577 (JP)
(74) Representative: Hoffmann Eitle
(86) International application number: PCT/JP2020/044312
(87) International publication number: WO 2021/107123

(57) **Abstract**

An object of the present invention is to provide a cell culture method having an excellent cell proliferation property, an antibody production method using the above method, an organic acid removal method using the above method, and an antibody produced by the above method. The cell culture method of the present invention is a cell culture method including a metabolic decomposition product removal step of bringing a culture solution containing a metabolic decomposition product into contact with a polymer having a quaternary ammonium base group or an amino group and removing the metabolic decomposition product from the culture solution to a purified culture solution and a cell culture step of culturing cells using the purified culture solution.

## Description

### BACKGROUND OF THE INVENTION

### 1. Field of the Invention

The present invention relates to a cell culture method, an antibody production method, an organic acid removal method, and an antibody

### 2. Description of the Related Art

In the production of the biopharmaceutical product, a method of culturing cells while removing ammonia, which is a metabolite of cells, from a culture solution is known in order to improve productivity (for example, JP1986-63284A (JP-S61-63284A)). Further, a method of removing a specific substance from a biological liquid such as blood or a culture solution is also disclosed in WO2016/013540A.

### SUMMARY OF THE INVENTION

In fed-batch culture, the accumulation of metabolic decomposition products secreted from cells may reduce the number of cells in the latter half of the culture, which results in an insufficient cell proliferation property. On the other hand, a perfusion culture method, in which the culture solution is continuously filtered and discharged for the purpose of removing these accumulated metabolic decomposition products, while the culture solution is continuously supplied to the culture vessel, is used. In the perfusion culture method, the number of cells is large as compared with that in the fed-batch culture; however, a large amount of culture solution is used, and thus there is a problem that the cost is high.

Therefore, in consideration of the above circumstances, an object of the present invention is to provide a cell culture method having an excellent cell proliferation property, an antibody production method using the above method, an organic acid removal method using the above method, and an antibody produced by the above method.

As a result of diligent studies on the above-described object, the inventors of the present invention have found that in a case where cells are cultured using a culture solution treated with a polymer having a quaternary ammonium base group or an amino group, the above object can be achieved, thereby reaching the present invention.

That is, the inventors of the present invention have found that the above-described object can be achieved by the following configurations.
(1) A cell culture method comprising:
   a metabolic decomposition product removal step of bringing a culture solution containing a metabolic decomposition product into contact with a polymer having a quaternary ammonium base group or an amino group and removing the metabolic decomposition product from the culture solution to a purified culture solution; and
   a cell culture step of culturing cells using the purified culture solution.
(2) The cell culture method according to (1), in which the culture solution containing the metabolic decomposition product is a culture solution taken out from the culture solution in the culture step.
(3) The cell culture method according to (1) or (2), in which the culture solution containing the metabolic decomposition product does not contain cells.
(4) The cell culture method according to (3), in which the culture solution containing the metabolic decomposition product is a culture solution obtained by filtering a culture solution taken out from the culture solution in the culture step.
(5) The cell culture method according to (4),
   in which the metabolic decomposition product removal step is a step of passing the filtered culture solution through a purification unit filled with the polymer and removing the metabolic decomposition product from the culture solution to obtain the purified culture solution,
   the culture step is a step of culturing cells using the purified culture solution in a culture vessel,
   an inlet side of the purification unit and the culture vessel are connected by a flow channel having a separation membrane in the middle of the flow channel, and
   the filtered culture solution is a culture solution obtained by filtering, with the separation membrane, the culture solution taken out from the culture solution in the culture step through the flow channel.
(6) The cell culture method according to any one of (1) to (5), further comprising a nutritional component addition step of adding a nutritional component to the purified culture solution obtained in the metabolic decomposition product removal step to obtain a purified culture solution to which the nutritional component has been added.
(7) The cell culture method according to (6), in which the nutritional component contains at least one selected from the group consisting of amino acids, vitamins, energy sources, osmotic pressure regulators, pH buffers, trace metal elements, surfactants, and growth supporting factors.
(8) The cell culture method according to(6) or (7), in which the nutritional component contains at least one selected from the group consisting of vitamins, energy sources, pH buffers, and trace metal elements.
(9) The cell culture method according to any one of (1) to (8), in which the metabolic decomposition product is an organic acid.
(10) The cell culture method according to (9), in which the organic acid includes at least any one of isovaleric acid, butyric acid, or citric acid.
(11) The cell culture method according to any one of (1) to (10), in which the polymer is a water-insoluble polymer having a quaternary ammonium base group.
(12) The cell culture method according to (11), in which the water-insoluble polymer has a partial structure represented by General Formula (p1) described later,
(13) The cell culture method according to (12), in which in General Formula (p1), R₁, R₂, and R₃ are each independently a methyl group or a hydroxyethyl group, and X is Cl, Br, OTs, or PF₆.
(14) The cell culture method according to (11), in which the water-insoluble polymer has a repeating unit represented by General Formula (P1) or (P2) described later,
(15) The cell culture method according to (14), in which the water-insoluble polymer has a repeating unit represented by General Formula (PI).
(16) The cell culture method according to (15), in which in General Formula (PI), R₁, R₂, and R₃ are a methyl group, and X is Cl or Br.
(17) The cell culture method according to any one of (1) to (16), in which the cell is a floating cell.
(18) The cell culture method according to any one of (1) to (17), in which the cell is an animal cell.
(19) The cell culture method according to any one of (1) to (18), in which the cell is a CHO cell.
(20) The cell culture method according to any one of (1) to (19), in which the cell is a productive cell.
(21) The cell culture method according to (20), in which the productive cell is an antibody-producing cell.
(22) An antibody production method using the cell culture method according to (21).
(23) An organic acid removal method, comprising removing at least one organic acid selected from the group consisting of isovaleric acid, butyric acid, and citric acid from a culture solution by using the cell culture method according to (19).

As described later, according to the present invention, it is possible to provide a cell culture method having an excellent cell proliferation property, an antibody production method using the above method, an organic acid removal method using the above method, and an antibody produced by using the above method.

### BRIEF DESCRIPTION OF THE DRAWINGS

Fig. 1 is a view schematically illustrating one aspect of a preferred aspect of the method of the present invention.
Fig. 2 is a view schematically illustrating one aspect of a preferred aspect of the method of the present invention (a combination with N-1 culture).
Fig. 3 is a view schematically illustrating one aspect of a preferred aspect of the method of the present invention (a combination with perfusion culture).
Fig. 4 is a table showing the molecular weight cut-off and the estimated value of the average pore diameter of the ultrafiltration membrane.

### DESCRIPTION OF THE PREFERRED EMBODIMENTS

Hereinafter, a cell culture method and an antibody production method according to the embodiment of the present invention will be described.

In the present invention, the numerical value range indicated by using "to" means a range including the numerical values before and after "to" as the lower limit value and the upper limit value, respectively.

It is noted that respective components may be used alone or in a combination of two or more kinds thereof. In a case where two or more kinds of respective components are used in combination, the content of thereof indicates a total content unless otherwise specified.

Further, in the present specification, in a case where the amount of the specific adsorbent with respect to the metabolic decomposition product-containing culture solution, which is indicated by using "% by mass", is an amount obtained by dividing the amount (polymer content, g) of the specific adsorbent by the amount (g) of the metabolic decomposition product-containing culture solution in a case where 1 ml of the culture solution is converted as 1 g.

### [1] Cell culture method

The cell culture method according to the embodiment of the present invention (hereinafter, also simply referred to as "the method according to the embodiment of the present invention") is a cell culture method including:
a metabolic decomposition product removal step of bringing a culture solution containing a metabolic decomposition product into contact with a polymer having a quaternary ammonium base group or an amino group and removing the metabolic decomposition product from the culture solution to a purified culture solution; and
a cell culture step of culturing cells using the purified culture solution.

Since the method according to the embodiment of the present invention has such a configuration, it is conceived that the above-described effects can be obtained. The reason for the above is not clear, but it is presumed to be as follows.

As described above, in the method according to the embodiment of the present invention, cells are cultured using a culture solution obtained by treating a culture solution containing metabolic decomposition products with a polymer having a quaternary ammonium base group or an amino group. Here, from the studies by the inventors of the present invention, it has been found that among the metabolic decomposition products, organic acids such as isovaleric acid, butyric acid, and citric acid inhibit the cell culture. It is presumed that as described above, in the method according to the embodiment of the present invention, the culture solution containing metabolic decomposition products is treated using a polymer having a quaternary ammonium base group or an amino group, and thus the metabolic decomposition products (particularly organic acids) in the culture solution are efficiently removed. That is, the polymer having a quaternary ammonium base group or an amino group is conceived to act as an extremely effective adsorbent with respect to metabolic decomposition products (particularly organic acids). As a result, it is presumed that the method according to the embodiment of the present invention in which cells are cultured using a culture solution (a purified culture solution) treated with such an adsorbent exhibits an excellent cell proliferation property.

In addition, in a case where cells are removed from the liquid after culturing, they are usually filtered with a depth filter, where clogging is also troublesome. Generally, in a case of culturing in an environment with a small amount of metabolic decomposition product, the proportion of dead cells among all cells (live cells + dead cells) decreases as compared with in a case of culturing in a state with a large amount of metabolic decomposition product. It is presumed that in a case where the culture solution of the culture using the present invention is used, clogging tends to occur more hardly during purification as compared with a case where it is not used. It is noted that unless otherwise specified in the present specification, cells mean live cells.

Hereinafter, each of the steps included in the method according to the embodiment of the present invention will be described.

### [Metabolic decomposition product removal step]

The metabolic decomposition product removal step is a step of bringing a culture solution containing a metabolic decomposition product into contact with a polymer having a quaternary ammonium base group or an amino group and removing the metabolic decomposition product from the culture solution to a purified culture solution having a small amount of metabolic decomposition product.

### [Culture solution containing metabolic decomposition products]

The culture solution used in the metabolic decomposition product removal step contains metabolic decomposition products. Hereinafter, the "culture solution containing metabolic decomposition products" is also referred to as a "metabolic decomposition product-containing culture solution".

### <Metabolic decomposition product>

The metabolic decomposition products are unnecessary substances that are produced at the time of culturing cells and include those produced by the cells. Examples thereof include organic acids and the like.

The metabolic decomposition product is preferably an organic acid and more preferably includes at least one of isovaleric acid, butyric acid, or citric acid, due to the reason that the effect of the present invention is more excellent.

The concentration of the isovaleric acid in the culture solution is not particularly limited; however, it is preferably 10 µM or more, more preferably 50 µM or more, and still more preferably 250 µM or more, due to the reason that the effects of the present invention are more excellent. In addition, it is preferably 10 µM or more and 10 mM or less, more preferably 50 µM or more and 5 mM or less, and still more preferably 250 µM or more and 5 mM or less, due to the reason that the effects of the present invention are more excellent.

The concentration of the butyric acid in the culture solution is not particularly limited; however, it is preferably 10 µM or more, more preferably 200 µM or more, and still more preferably 350 µM or more, due to the reason that the effects of the present invention are more excellent. In addition, it is preferably 10 µM or more and 100 mM or less, more preferably 200 µM or more and 20 mM or less, and still more preferably 350 µM or more and 20 mM or less, due to the reason that the effects of the present invention are more excellent.

The concentration of the citric acid in the culture solution is not particularly limited; however, it is preferably 50 µM or more, more preferably 500 µM or more, and still more preferably 800 µM or more, due to the reason that the effects of the present invention are more excellent. In addition, it is preferably 50 µM or more and 500 mM or less, more preferably 500 µM or more and 100 mM or less, and still more preferably 800 µM or more and 100 mM or less, due to the reason that the effects of the present invention are more excellent.

### <Culture solution>

The culture solution is a liquid containing substances necessary for cell growth, and a culture solution for cell culture, which is used in the related art, can be appropriately used. The culture solution contains, for example, amino acids, vitamins, lipid factors, an energy source, an osmotic pressure regulator, an iron source, and a pH buffer. In addition to the above components, it may contain, for example, a trace metal element, a surfactant, a growth supporting factor, and a nucleoside.

It suffices that the culture solution is fluid, including not only liquid but also sol. A commercially available culture medium for culturing animal cells, for example, CD OptiCHO, a BME culture medium, an MEM culture medium, a DMEM culture medium, an F10 culture medium, or an F12 culture medium also suitably used, but it is not limited thereto.

### <Preferred aspect of the metabolic decomposition product-containing culture solution>

The metabolic decomposition product-containing culture solution is preferably a culture solution taken out from the culture solution in the culture step described later, due to the reason that the effects of the present invention are more excellent.

The metabolic decomposition product-containing culture solution preferably does not contain cells due to the reason that the effects of the present invention are more excellent. In a case where cells are not contained, cell viability can be improved.

The metabolic decomposition product-containing culture solution is preferably a culture solution obtained by filtering a culture solution taken out from the culture solution in the culture step described later, due to the reason that the effects of the present invention are more excellent.

Since the culture solution in the culture step described later usually contains cells, the cells can be removed by filtering. Filtration can be carried out, for example, by using a separation membrane.

### [Specific adsorbent]

As described above, in the metabolic decomposition product removal step, the metabolic decomposition product-containing culture solution is brought into contact with a polymer having a quaternary ammonium base group or an amino group. As a result, metabolic decomposition products (particularly organic acids) are adsorbed to the polymer having a quaternary ammonium base group or an amino group, and a culture solution (a purified culture solution) having a small amount of metabolic decomposition product can be obtained. That is, the polymer having a quaternary ammonium base group or an amino group acts as an extremely effective adsorbent. Hereinafter, the "polymer having a quaternary ammonium base group or an amino group" is also referred to as a "specific adsorbent".

### <Water-insoluble polymer>

The specific adsorbent is preferably a water-insoluble polymer having a quaternary ammonium base group or an amino group, and more preferably a water-insoluble polymer having a quaternary ammonium base group, due to the reason that the effects of the present invention are more excellent.

The water-insoluble polymer is preferably water-insoluble at 37°C due to the reason that the effects of the present invention are more excellent. Here, the term "water insoluble" means that 1 g of the polymer is dissolved in 100 g of water at 37°C, filtered, and then the water is volatilized to obtain a water content of 0.05 g or less.

The shape of the water-insoluble polymer is not particularly limited, and it may be any one of a particle shape, a hollow fiber shape, a non-woven fabric shape, a fibrous shape, a sheet shape, a mesh shape, a porous shape, a sponge shape, or an irregular shape. In order to improve the efficiency of purification, a shape by which the surface area increases is good, and thus a porous shape is preferable.

The water-insoluble polymer may be combined with a carrier. In a case where the specific adsorbent contains a water-insoluble polymer and a carrier, the carrier may contain activated carbon, polyester, or polysulfone.

### <Functional group>

The specific adsorbent is not particularly limited as long as it is a polymer having a quaternary ammonium base group or an amino group; however, it is preferably a polymer having a quaternary ammonium base group due to the reason that the effects of the present invention are more excellent.

The quaternary ammonium base group is preferably a trialkyl ammonium base group or a dialkylhydroxyethyl ammonium base group, more preferably a trialkyl ammonium base group, and still more preferably a trimethyl ammonium base group, due to the reason that the effects of the present invention are more excellent.

### <Main chain>

The main chain of the specific adsorbent is not particularly limited; however, it is preferably acrylic or styrene-based, and more preferably styrene-based, due to the reason that the effects of the present invention are more excellent.

### <Anion>

In a case where the specific adsorbent is a polymer having a quaternary ammonium base group, the counter anion is not particularly limited; however, it is preferably OH, Cl, Br, OTs, or PF₆, more preferably Cl or Br, and still more preferably Cl, due to the reason that the effects of the present invention are more excellent. Here, Ts represents CH₃-C₆H₄-SO₂-.

### <Preferred aspect of specific adsorbent>

The specific adsorbent is preferably a water-insoluble polymer having a quaternary ammonium base group due to the reason that the effects of the present invention are more excellent.

The water-insoluble polymer having a quaternary ammonium base group is more preferably a water-insoluble polymer having a partial structure represented by General Formula (p1) due to the reason that the effects of the present invention are more excellent.

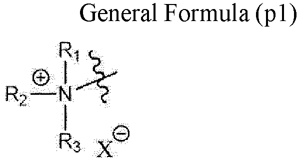

In General Formula (p1), R₁, R₂, and R₃ each independently represent a hydrogen atom, an alkyl group having 1 to 12 carbon atoms, or a phenyl group, and may have a hydroxyl group as a substituent. X represents OH, Cl, Br, OTs, or PF₆. Here, Ts represents CH₃-C₆H₄-SO₂-.

In General Formula (p1), R₁, R₂, and R₃ are each independently preferably a methyl group or a hydroxyethyl group due to the reason that the effects of the present invention are more excellent.

In General Formula (p1), X is preferably Cl, Br, OTs, or PF₆ due to the reason that the effects of the present invention are more excellent.

The water-insoluble polymer having a quaternary ammonium base group preferably has a repeating unit represented by General Formula (P1) or (P2) and more preferably has a repeating unit represented by General Formula (P1) due to the reason that the effects of the present invention are more excellent.

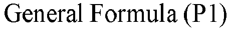

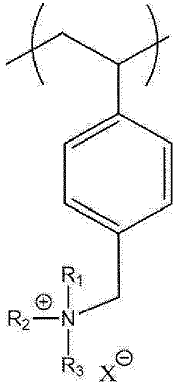

In General Formula (PI), R₁, R₂, and R₃ each independently represent a hydrogen atom, an alkyl group having 1 to 12 carbon atoms, or a phenyl group. X represents OH, Cl, Br, OTs, or PF₆. Here, Ts represents CH₃-C₆H₄-SO₂-.

In General Formula (PI), R₁, R₂, and R₃ are preferably a methyl group due to the reason that the effects of the present invention are more excellent.

In General Formula (PI), X is preferably Cl or Br due to the reason that the effects of the present invention are more excellent.

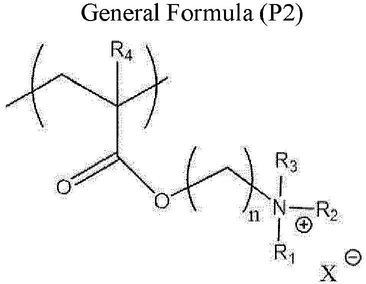

In General Formula (P2), R₁, R₂, R₃, and R₄ each independently represent a hydrogen atom, an alkyl group having 1 to 12 carbon atoms, or a phenyl group. n represents an integer of 1 to 8. X represents OH, Cl, Br, OTs, or PF₆. Here, Ts represents CH₃-C₆H₄-SO₂-.

The specific adsorbent is preferably one that does not interfere with cell proliferation due to the reason that the effects of the present invention are more excellent.

It is better for the specific adsorbent not to adsorb those (nutritional components) that are used for cell nutrition due to the reason that the effects of the present invention are more excellent. Specific examples of the nutritional components are as described below.

As the surface area of the specific adsorbent becomes large, the adsorption rate of the organic acid is improved, and thus the using amount of the specific adsorbent becomes small, which is preferable in terms of cost. However, the same effect can be obtained by increasing the using amount even in a case where the specific adsorbent has a small surface area.

The surface area of the adsorbing material refers to a surface area calculated using the BET (JIS Z 8830; specific surface area measurement method for powder (solid) by gas adsorption) analysis method after measuring the nitrogen adsorption isotherm at the liquid nitrogen temperature (77K). There are two methods of measuring the nitrogen adsorption isotherm, a volumetric method of calculating the amount of nitrogen adsorbed to the carrier by reducing the pressure of the nitrogen gas filled in the cell and a gravimetric method of measuring the weight change of the carrier, either of which may be used for the measurement. Any method can be used as long as it can measure the surface area.

### <Amount of specific adsorbent>

The amount of the specific adsorbent with respect to the metabolic decomposition product-containing culture solution is preferably 0.5% to 40% by mass, more preferably 1.0% to 30% by mass, still more preferably 2.0% to 20% by mass, and particularly preferably 2.0% to 10% by mass, due to the reason that the effects of the present invention are more excellent.

The amount of the specific adsorbent with respect to the culture solution that is used in the culture step described later is preferably 1% by mass or more, more preferably 2% by mass or more, and still more preferably 4% by mass or more, due to the reason that the effects of the present invention are more excellent.

### [Method of bringing into contact]

The method of bringing the metabolic decomposition product-containing culture solution into contact with the specific adsorbent is not particularly limited, and examples thereof include a method of mixing and stirring both of them and a method of passing the metabolic decomposition product-containing culture solution through a purification unit filled with the specific adsorbent. For example, the specific adsorbent may be brought into contact with the stored metabolic decomposition product-containing culture solution, or an adsorbent may be brought into contact with the flowing metabolic decomposition product-containing culture solution.

It is preferable that the specific adsorbent does not come out from the purification unit, and in that case, it is preferable that the specific adsorbent is fixed or that the specific adsorbent is prevented from coming out from the purification unit with an isolation filter (a filter that isolates the specific adsorbent) such as a glass filter. In a case of a single flow pump, it may be sufficient to install an isolation filter, for example, only in the direction of the flow. However, in a case of using an alternating tangential flow (ATF) pump, it is preferable to install isolation filters both on the inlet side and the outlet side of the purification unit.

The specific adsorbent may be produced by chemical synthesis or may be purchased. For example, it can be purchased from Mitsubishi Chemical Corporation, ORGANO Corporation, or Purolite.

### [Preferred aspect of metabolic decomposition product removal step]

It is preferable that the specific adsorbent does not come into direct contact with cells in the metabolic decomposition product removal step due to the reason that the effects of the present invention are more excellent.

The fact that the specific adsorbent does not come into direct contact with cells is not limited to the fact that the specific adsorbent does not come into direct contact with cells, and it is sufficient to take measures so that the specific adsorbent does not come into contact with cells. For example, in a case where the specific adsorbent is installed in the culture vessel of the culture step described later, the periphery of the specific adsorbent can be covered with a mesh or an isolation membrane such as a microfiltration membrane or an ultrafiltration membrane. The isolation membrane is also called a separation membrane. In addition, a flow channel is provided in the culture vessel, and a separation membrane unit (a unit having a separation membrane capable of separating cells) is provided in the middle of the flow channel. Here, the separation membrane unit is preferably set on the culture container (the culture vessel) side of the flow channel for the purpose of separating cells. Then, it is also possible to provide a purification unit and bring the culture solution that has passed through the separation membrane unit in the purification unit into contact with the specific adsorbent. Further, it is also possible to take out the culture solution from the culture system, treat it with the specific adsorbent to remove metabolic decomposition products (particularly organic acids), and then return again the treated culture solution to the culture system.

The membrane separation treatment by the tangential filtration method is an effective treatment for reducing the damage to cells and suppressing the decrease in the permeation rate of the product. In a case where the membrane separation treatment by the tangential filtration method is employed, it is possible to reduce damage caused by cells being strongly pressed against a membrane, compared with a case where the membrane separation treatment is carried out by a dead-end method.

In the separation treatment step in the cell culture method according to the embodiment of the present invention and the production method for a product (for example, an antibody) using the cell culture method according to the embodiment of the present invention (hereinafter collectively referred to as the "production method for a product according to the present disclosure"), it is preferable that a cell suspension (a culture solution containing cells) is extracted from the culture container and separated, using the separation membrane, into a return solution having a higher cell concentration (a cell density) than the cell suspension and a permeated solution containing almost no cells by a tangential filtration method; however, it is not limited thereto. The tangential filtration method is a method in which by flowing a liquid to be subjected to the membrane separation treatment along a membrane surface of the separation membrane, components having a small size and liquid components are moved to a permeation side of the separation membrane, and components having a large size and remaining liquid components are retained on a non-permeation side of the separation membrane. In the present disclosure, the non-permeation side of the separation membrane, that is, the supply side is also referred to as a primary side, and the permeation side of the separation membrane is also referred to as a secondary side.

In the membrane separation treatment by the tangential filtration method in the present disclosure, flow of the cell suspension extracted from the culture container in one direction parallel to the membrane surface of the separation membrane may be formed, or reciprocating flow may be formed by reversing the flow direction of the cell suspension extracted from the culture container every predetermined time.

In the production method for a product according to the present disclosure, the term "step" in the step of culturing cells, the step of carrying treatment with the adsorbent, the step of returning the return solution to the culture container, and the step of recovering the product includes not only a treatment that is sequentially carried out batchwisely (for example, one treatment is completed, and then the next treatment is started) but also a treatment that is carried out in parallel in terms of time. Therefore, it also includes, for example, a case where a cell suspension is extracted from the culture container to carry out the separation treatment step while carrying out the culture step. Moreover, it also includes a case where the feeding of the return solution to the culture container, the supply of the fresh culture medium to the culture container, and the recovery of the product are also carried out in parallel with the culture step and the separation treatment step in terms of time. In a case where the respective steps are carried out in parallel in terms of time, a produced product can be efficiently separated and recovered. Furthermore, the respective steps may be continuously carried out, but for example, there may be a temporary stop time for switching a flow direction of tangential filtration in the separation treatment step.

The extraction of the cell suspension from the culture container can usually be carried out using a pump, but other available liquid feeding units may be used. The cell suspension extracted from the culture container is fed to a separation membrane unit. The extraction of the cell suspension from the culture container to the separation membrane unit may be carried out intermittently or continuously; however, it is preferably carried out continuously from the viewpoint that a system state is stably maintained.

The separation membrane unit includes, for example, a container and a separation membrane, and the separation membrane separates a space inside the container into a supply side and a permeation side and carries out a membrane separation treatment on the cell suspension extracted from the culture container. On the supply side of the separation membrane, the separation membrane unit has an in-flow port and an out-flow port, each of which is connected to a pipe. In the membrane separation treatment by the tangential filtration method, in a case where the flow direction of the cell suspension extracted from the culture container is reversed every predetermined time, the in-flow port and the out-flow port are switched by reversing the flow direction.

As the separation membrane, a mesh filter formed by weaving fibrous members in a mesh shape can be used. In a case of using the mesh filter, it is possible to promote the discharge of components unnecessary for cell culture, which includes dead cell bodies and cell debris, to the permeation side, as compared with a case of using a hollow fiber membrane. As a result, components unnecessary for cell culture can be effectively removed from the culture container.

In addition, as the separation membrane, a hollow fiber membrane can be used. In a case where a hollow fiber membrane is used, the risk of the cell permeation into the permeation side can be reduced as compared with a case of using the mesh filter. Moreover, the risk of occurrence of clogging due to the entry of cells into the separation membrane can be reduced. This makes it possible to reduce the loss of cells. In this case, as the hollow fiber membrane, a microfiltration membrane (an MF membrane) or an ultrafiltration membrane (a UF membrane) can be used; however, it is not limited thereto.

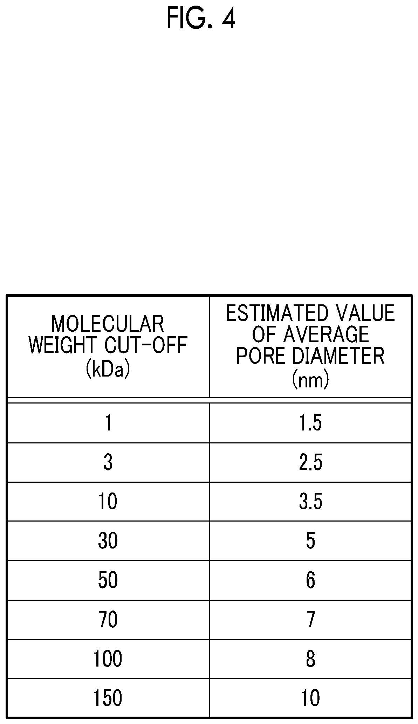

In a case where the separation membrane is a microfiltration membrane (an MF membrane) or an ultrafiltration membrane (a UF membrane), the pore diameter Dp of the separation membrane can be measured as the average pore diameter by a mercury press-in method. For example, the pore diameter Dp can be measured by pressing mercury into the separation membrane with high pressure by using AUTOPORE IV 9520 manufactured by Shimadzu Corporation. It is noted that the ultrafiltration membrane (the UF membrane) is a filtration membrane having a smaller average pore diameter than the microfiltration membrane (also referred to as an MF membrane), where the average pore diameter thereof is 0.001 to 0.01 µm and the separation performance thereof is defined by the molecular weight cut-off. In the ultrafiltration membrane having a small pore diameter, the measurement of the average pore diameter by a mercury press-in method may be difficult. In this case, the average pore diameter of the ultrafiltration membrane can be estimated based on the molecular weight cut-off. Specifically, as described in http://chemeng.in.coocan.jp/memb/m_mb4.html, a molecular weight of a standard substance, which is obtained in a case where a blocking rate is 90% after permeation of a plurality of kinds of standard substances having known molecular weights through a target ultrafiltration membrane, is defined as a molecular weight cut-off of this ultrafiltration membrane. Since the molecular weight can be estimated from a molecular weight of a standard substance, the average pore diameter of the ultrafiltration membrane can be estimated based on a molecular weight cut-off. Specifically, as described in Fig. 4, the average pore diameter is estimated from the molecular weight cut-off. In a case where the molecular weight cut-off does not match a molecular weight of a standard substance, the average pore diameter can be estimated by plotting the molecular weight cut-off and the average pore diameter and carrying out interpolation by linear interpolation or the like.

From the viewpoint of suppressing the permeation of cells which produce a product, the pore diameter Dp of the separation membrane is preferably 1 µm or less, more preferably 0.8 µm or less, still more preferably 0.23 µm or less, and most preferably 0.2 µm or less. Further, in this method, it is preferable to use an MF membrane or a UF membrane as the separation membrane, and it is still more preferable to use a UF membrane, due to the reason that the effects of the present invention are more excellent.

The molecular weight cut-off of the UF membrane to be used is preferably 100 kDa or less, more preferably 0.1 kDa to 50 kDa, still more preferably 0.1 kDa to 10 kDa, and most preferably 1 kDa to 5 kDa.

The separation membrane does not allow live cells in the cell suspension to permeate, but it allows metabolic decomposition products such as isovaleric acid, butyric acid, and citric acid to permeate. The reason why the difference in permeability occurs between the live cells and the product is that there is a difference between the size of the live cell and the size of the metabolic decomposition product that is desired to be removed. For example, in a case where an antibody is produced in animal cells, the molecular weight of the metabolic decomposition product is 1 kD or less, and the estimated value of the average pore diameter of the ultrafiltration membrane is 1.5 nm or less from Fig. 4, whereas the order of magnitude of the size of the animal cell is 10 in terms of µm. For this reason, for example, in a case where a microfiltration membrane or an ultrafiltration membrane is used as the separation membrane, the metabolic decomposition product can be transferred to the secondary side through the separation membrane while keeping the live cells on the primary side. Further, since the size of the liquid component, for example, a water molecule is very small, a part of the liquid component also transfers to the secondary side together with the antibody. As a result, the cell concentration in the liquid remaining on the primary side is increased. As described above, by carrying out the separation by the tangential filtration method, the cell suspension fed to the separation membrane unit is separated into a return solution which remains on the primary side without permeating the separation membrane and has a cell concentration higher than that of the cell suspension and a permeated solution which permeates the separation membrane to be transferred to the secondary side and has a cell concentration lower than that of the cell suspension. The cell concentration in the cell suspension referred to here means the live cell concentration in the cell suspension in the culture container. Further, in the present disclosure, the "cell concentration" refers to the "live cell concentration" unless otherwise particularly specified.

The return solution remaining on the primary side flows out from the separation membrane unit, passes through the column containing the adsorbent, and returns to the culture container. For example, in a case where the direction of flow in the space on the supply side of the separation membrane unit in the tangential filtration is fixed in one direction, the cell suspension may be returned to the culture container through a pipe that connects the out-flow port, the column containing the adsorbent, and the culture container, by reducing the pressure from the secondary side by a pump different from the pump by which the supply is carried out from the culture container to the separation membrane unit and drawing the liquid. In this case, circulating flow is formed by returning the return solution to the culture container through a pipe different from a pipe through which the cell suspension in the culture container is moved to the in-flow port of the separation membrane unit. As a result, even a case where the direction of flow in the space on the supply side of the separation membrane unit in the tangential flow filtration is reversed along with the passage of time, that is, even in a case where the flow is reciprocated, it is possible to fix the direction of flow in the space on the supply side of the separation membrane unit in one direction by reducing the pressure from the secondary side by a pump in the same manner.

In a case where the circulating flow is formed, for example, KrosFlo perfusion culture flow path device (KML-100, KPS-200, or KPS-600) of Spectrum Laboratories Inc. or PuraLev series manufactured by Levitronix can be suitably used. Moreover, in a case where the reciprocating flow is formed, an ATF system of Repligen Corporation can be suitably used.

The rate at which the liquid is fed to the adsorbent column (the purification unit) by using the separation membrane is preferably 0.1 vvd or more and 100 vvd or less, and more preferably 0.3 vvd or more and 20 vvd or less. Here, vvd is a unit that indicates an amount obtained by dividing the liquid amount passing through the column per day by the total amount of the culture vessel liquid.

### [Purified culture solution]

As described above, in the metabolic decomposition product removal step, a purified culture solution having a small amount of metabolic decomposition product can be obtained.

It suffices that the purified culture solution has a reduced concentration of metabolic decomposition products (for example, isovaleric acid, butyric acid, and citric acid). However, due to the reason that the effects of the present invention are more excellent, it is preferable that the concentration of isovaleric acid is 200 µM or less, the concentration of butyric acid is 400 µM or less, and the concentration of citric acid is 900 µM or less, and it is more preferable that the concentration of isovaleric acid is 150 µM or less, the concentration of butyric acid is 200 µM or less, and the concentration of citric acid is 500 µM or less.

### [Culture step]

The culture step is a step of culturing cells using the purified culture solution obtained in the above-described metabolic decomposition product removal step. In the culture step, a part or the whole of the above-described purified culture solution may be used. In the culture step, another culture solution may be used in addition to the above-described purified culture solution.

It is noted that the cell culture means separating cells from a microorganism, an animal, or a plant, and proliferating and maintaining them in vitro. The process of separating cells from a living body and carrying out the first replating is called primary culture, and the process of transferring existing cultured cells to a new culture container and proliferating and maintaining them is called subculture.

### [Cell]

The cell is not particularly limited, and it may be a prokaryotic cell such as Escherichia coli, yeast, a plant cell, an animal cell, or Bacillus subtilis. It is preferably an animal cell, more preferably a mammalian cell, still more preferably, a baby hamster kidney (BHK) cell, a 293 cell, a Chinese hamster ovary (CHO) cell, an NS0 cell, an SP2/0 cell, or a hybridoma cell, and particularly preferably a CHO cell, due to the reason that the effects of the present invention are more excellent.

The cell is preferably a floating cell due to the reason that the effects of the present invention are more excellent.

The cell is preferably a productive cell.

The product of the cell is not particularly limited as long as it is produced by the cell; however, it may be a deoxyribonucleic acid, a ribonucleic acid, a protein, a hormone, a cytokine, a virus, an alcohol, an enzyme, an antibiotic, or a recombinant protein, and it is preferably a protein and more preferably an antibody. That is, the cell is preferably an antibody-producing cell.

The antibody to be produced by the animal cell is not particularly limited; however, examples thereof include an anti-IL-6 receptor antibody, an anti-IL-6 antibody, an anti-glypican-3 antibody, an anti-CD3 antibody, an anti-CD20 antibody, an anti-GPIIb/IIIa antibody, an anti-TNF antibody, an anti-CD25 antibody, an anti-EGFR antibody, an anti-Her2/neu antibody, an anti-RSV antibody, an anti-CD33 antibody, an anti-CD52 antibody, an anti-IgE antibody, an anti-CD11a antibody, an anti-VEGF antibody, and an anti-VLA4 antibody. The antibody includes not only a monoclonal antibody derived from an animal such as a human being, a mouse, a rat, a hamster, a rabbit, and a monkey but also an artificially modified antibody such as a chimeric antibody, a humanized antibody, and a bispecific antibody.

### [Method of culturing cell]

The method of culturing cells is not particularly limited; however, it is preferably fed-batch culture, batch culture, or perfusion culture (perfusing), and particularly preferably fed-batch culture due to the reason that the effects of the present invention are more excellent.

The above-described fed-batch culture is also called feeding culture or fed batch culture, and it is a culture method that is carried out so that a microorganism or animal or plant cells are not removed from a bioreactor until the time of harvest in a case of culturing the microorganism or the cells in the bioreactor (the culture vessel) while supplying a certain specific nutrient source or a culture solution component during the culture. Since the fed-batch culture is a culture method in which a large amount of metabolic decomposition product is accumulated, the effects of the present invention are particularly excellent.

As described above, it is also possible to carry out fed-batch culture and perfusion culture, as well as a culture obtained by combining with N-1 culture. Here, the perfusion culture in the present specification is a culture method in which a new culture medium is continuously supplied while cells remain in the culture solution, and culture is carried out while the old culture medium is continuously discarded. In addition, the N-1 culture shall be a culture method in which perfusion culture is carried out in the first half, and then at a stage where the number of cells is sufficiently increased, the supply of the new culture medium and the discarding of the old culture medium are stopped, and the culture is shifted to fed-batch culture.

The concentration of seeded cells and the maximum cell concentration in the culture can be determined by measuring the number of cells by a conventional method and dividing the number of cells by the amount of the culture solution. The maximum live cell concentration is the maximum value of the live cell concentration during the culture period, and varies depending on culture conditions.

The live cell rate (the survival rate) during the culture period is obtained by dividing the number of live cells by (the number of live cells + the number of dead cells). For example, the number of live cells and the number of dead cells can be measured using Vi-CELL XR (product name, Beckman Coulter, Inc.) which uses viability determination by a trypan blue staining method.

The product to be targeted is contained in the culture solution after culturing. The product in the permeated solution can be recovered and used for various purposes such as the production of pharmaceutical products and foods. For example, the product can be purified by a purification treatment. The obtained product can be purified to high purity. In a case where the product is a polypeptide such as an antibody or a fragment thereof, the separation and purification of the product may be carried out by using the ordinary separation and purification method used for a polypeptide. For example, a chromatography column for affinity chromatography or the like, a filter, ultrafiltration, salting out, dialysis, SDS polyacrylamide gel electrophoresis, and isoelectric focusing electrophoresis can be appropriately selected and combined to separate and purify a polypeptide; however, the present invention is not limited to thereof. The concentration of the obtained polypeptide can be measured by measurement of absorbance, enzyme-linked immunosorbent assay (ELISA), or the like.

Examples of the column that is used for affinity chromatography include a protein A column and a protein G column. Examples of the chromatography other than affinity chromatography include ion exchange chromatography, hydrophobic chromatography, gel filtration, reverse phase chromatography, adsorption chromatography. The chromatography can be carried out using liquid phase chromatography such as high performance liquid chromatography (HPLC) or fast protein liquid chromatography (FPLC).

It is preferable to supply the feed into the culture container as a method of adding nutrients for continuing the growth of cells. In a case where the feed is supplied into the culture container, the state of cells in the culture container can be kept in a healthy state. This supply may be carried out intermittently or continuously. Further, the cell suspension volume in the culture container is not necessary to be perfectly constant at all times and may be substantially constant in a range of a variation of, for example, ±10%, ±5%, and ±1%. The measurement of the cell suspension volume in the culture container is not necessary to be carried out at all times and may be carried out intermittently.

In the production method for a product according to the present disclosure, the step of culturing a cell which produces a product may include stirring the cell culture solution in the culture container with a stirring blade.

In a case where the stirring blade is rotated, the cell suspension accommodated in the culture container is stirred, and thus homogeneity of the cell suspension is improved.

In the production method for a product according to the present disclosure, the step of culturing a cell that produces a product may include passing a gas through the cell suspension with a sparger.

### <Seeded cell density>

The seeded cell density (the initial cell density in the culture solution) (the initial number of cells) is preferably 0.1 × 10⁶ cells/mL to 1 × 10⁷ cells/mL, more preferably 0.5 × 10⁶ cells/mL to 2.5 × 10⁶ cells/mL, and still more preferably 0.5 × 10⁶ cells/mL to 2.0 × 10⁶ cells/mL, due to the reason that the effects of the present invention are more excellent.

### <Temperature and the like>

The temperature at which cells are cultured is preferably 29°C or higher and 40°C or lower, and more preferably 35°C or higher and 38°C or lower, due to the reason that the effects of the present invention are more excellent. The temperature may be changed during the culture period.

The culture is preferably carried out in an atmosphere having a carbon dioxide concentration of 0% to 40%, and preferably 2% to 10%.

### <Period>

The period during which cells are cultured is preferably 0 days or more and less than 50 days, more preferably 7 days or more and less than 40 days, and still more preferably 10 days or more and less than 25 days, due to the reason that the effects of the present invention are more excellent.

### <Culture scale>

The culture scale can be suitably selected in a case where it is 0.001 L or more. However, the culture scale is preferably 0.1 L or more, more preferably 1 L or more, still more preferably 50 L or more, and even still more preferably 300 L or more, due to the reason that from the viewpoint of saving the culture solution, the cost reduction effect is higher in a case where the scale is large and that the effects of the present invention are more excellent. The culture scale is preferably 0.1 L or more and 25,000 L or less, preferably 1 L or more and 25,000 L or less, more preferably 10 L or more and 5000 L or less, and still more preferably 40 L or more and 3000 L or less, due to the reason that the effects of the present invention are more excellent.

### [Nutritional component addition step]

Due to the reason that the effects of the present invention are more excellent, the method according to the embodiment of the present invention preferably further includes a nutritional component addition step of adding a nutritional component to the purified culture solution obtained in the above-described metabolic decomposition product removal step (for example, after the above-described metabolic decomposition product removal step and before the above-described culture step).

The above-described nutritional component is a substance necessary for cell growth, which is contained in the culture solution, where a nutritional component described later can be appropriately used, and examples thereof include sodium pyruvate, folic acid, pantothenic acid, pyridoxine hydrochloride, and biotin. The nutritional component preferably has a carboxylic acid group; however, it is not limited thereto.

The above-described nutritional component to be added is a substance necessary for cell growth, which is contained in the culture solution, and each of the components normally used in an animal cell culture medium can be appropriately used. It preferably contains at least one or more of an amino acid, vitamins, a lipid factor, an energy source, an osmotic pressure regulator, a pH buffer, a trace metal element, a surfactant, a growth supporting factor, a nucleoside, or salts.

Specifically, for example, the following components may be added: amino acids such as L-alanine, L-arginine, L-aspartic acid, L-aspartic acid, L-cysteine, L-cystine, L-glutamine, L-glutamic acid, glycine, L-histidine, L-isoleucine, L-leucine, L-lysine, L-methionine, L-ornithine, L-phenylalanine, L-proline, L-serine, L-threonine, L-tryptophan, L-tyrosine, and L-valine, and preferably amino acids such as L-alanine, L-arginine, L-asparagine, L-aspartic acid, L-cystine, L-glutamine, L-glutamic acid, glycine, L-histidine, L-isoleucine, L-leucine, L-lysine, L-methionine, L-phenylalanine, L-proline, L-serine, L-threonine, L-tryptophan, L-tyrosine, L-valine; vitamins such as inositol, biotin, folic acid, lipoic acid, nicotine amide, nicotinic acid, p-aminobenzoic acid, calcium pantothenate, pyridoxal hydrochloride, pyridoxine hydrochloride, riboflavin, thiamine hydrochloride, vitamin B12, and aspartic acid, and preferably biotin, folic acid, lipoic acid, nicotinic acid amide, calcium pantothenate, pyridoxal hydrochloride, riboflavin, thiamine hydrochloride, vitamin B12, and aspartic acid; lipid factors such as choline chloride, choline tartrate, linoleic acid, oleic acid, and cholesterol; energy sources such as glucose, galactose, mannose, fructose, and sodium pyruvate; osmotic pressure regulators such as sodium chloride, potassium chloride, and potassium nitrate; pH buffers such as sodium hydrogen carbonate, calcium chloride, or phosphate salts such as sodium dihydrogen phosphate and sodium monohydrogen phosphate, HEPES, and MOPS; trace metal elements such as salts of iron ions, such as EDTA iron, iron citrate, ferrous chloride, ferric chloride, ferrous sulfate, ferric sulfate, and ferrous nitrate, a copper ion, a manganese ion, a zinc ion, a magnesium ion, a magnesium ion, a nickel ion, a tin ion, and salts thereof such as copper sulfate, manganese sulfate, zinc sulfate, magnesium sulfate, nickel chloride, tin chloride, magnesium chloride, and sodium silicate; surfactants such as Tween 80 and Pluronic F68; and recombinant insulin, recombinant IGF, recombinant EGF, recombinant FGF, recombinant PDGF, recombinant TGF-α, ethanolamine hydrochloride, sodium selenite, retinoic acid, putrescine hydrochloride, growth supporting factors consisting of a polysulfated compound such as dextran sulfate or a salt thereof, heparan sulfate or a salt thereof, chondroitin sulfate or a salt thereof, dermatan sulfate or a salt thereof, pentosan sulfate or a salt thereof, or another proteoglycan or a salt thereof, and a polysulfonic acid compound; nucleosides such as deoxyadenosine, deoxycytidine, deoxyguanosine, adenosine, cytidine, guanosine, uridine.

The above-described preferred example of the present invention may contain an antibiotic such as streptomycin, penicillin G potassium, or gentamicin, a pH indicator such as phenol red, and a fish meat extract or enzymatic decomposition product of the fish meat.

It is still more preferable to contain at least one or more of amino acids, vitamins, an energy source, an osmotic pressure regulator, a pH buffer, a trace metal element, a surfactant, a growth supporting factor, it is even still more preferable to contain at least one or more selected from the group consisting of vitamins, energy sources, pH buffers, and a trace metal element, and it is most preferable to contain at least one or more of vitamins and a trace metal element.

In addition, it is preferable to add lactic acid as a nutrient. It is preferable to add 0.05 to 2.0 g of lactic acid per 1 L of the culture vessel, more preferably 0.1 to 1.0 g, and most preferably 0.2 to 1.0 g.

### [Preferred aspect of method according to embodiment of present invention]

Due to the reason that the effects of the present invention are more excellent, the method of the present invention is preferably a cell culture method,
where the above-described culture solution containing the metabolic decomposition product is a culture solution obtained by filtering a culture solution taken out from the above-described culture solution in the culture step,
the above-described metabolic decomposition product removal step is a step of passing the filtered culture solution through a purification unit filled with the above-described specific adsorbent and removing the metabolic decomposition product from the filled culture solution to obtain the above-described purified culture solution,
the above-described culture step is a step of culturing cells using the above-described purified culture solution in a culture vessel,
an inlet side of the purification unit and the culture vessel are connected by a flow channel having a separation membrane in the middle of the flow channel, and
the filtered culture solution is a culture solution obtained by filtering, with the separation membrane, the culture solution taken out from the culture solution in the culture step through the flow channel.

Hereinafter, the preferred aspect will be specifically described with reference to the drawings.

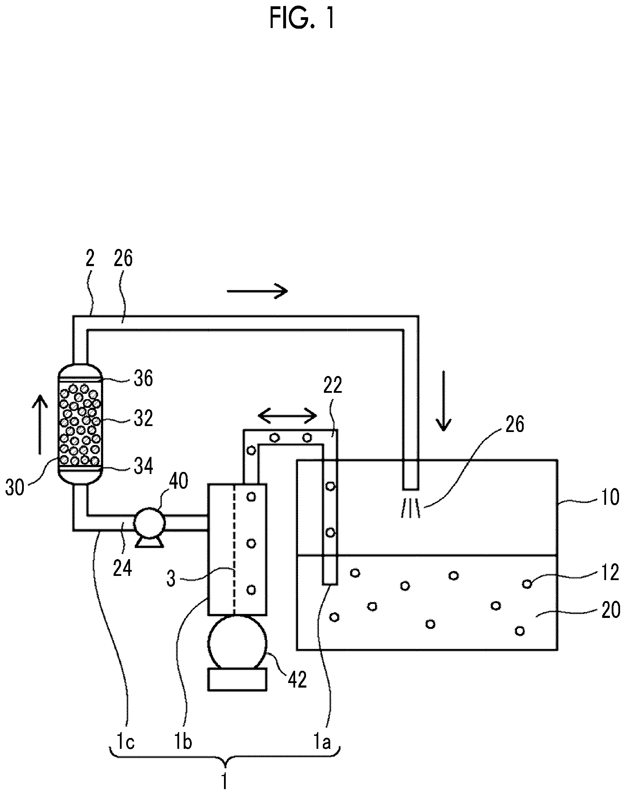

Fig. 1 is a view schematically illustrating one aspect of a preferred aspect of the method according to the embodiment of the present invention.

In Fig. 1, a culture solution 20 containing cells 12 is present in a culture vessel 10. The culture solution 20 contains metabolic decomposition products (not illustrated in the drawing) produced by culturing the cells 12.

Further, in Fig. 1, the inlet side of a purification unit 30 and the culture vessel 10 are connected by a flow channel 1. The purification unit 30 is filled with a specific adsorbent 32 described above. Further, the purification unit 30 has a filter 34 (on the inlet side) and a filter 36 (on the outlet side) so that the specific adsorbent 32 does not flow out from the purification unit 30. Here, the flow channel 1 consists of a flow channel 1a, a separation membrane unit 1b, and a flow channel 1c, the inlet side of the flow channel 1a is immersed in the culture solution 20 of the culture vessel 10, the outlet side of the flow channel 1 is connected to the inlet side of the separation membrane unit 1b, the outlet side of the separation membrane unit 1b is connected to the inlet side of the flow channel 1c, and the outlet side of the flow channel 1c is connected to the inlet side of the purification unit 30. Further, the separation membrane unit 1b has a separation membrane 3 between the inlet side thereof and the outlet side thereof. The separation membrane 3 is a membrane capable of separating the culture solution and cells so that the cells do not pass through thereto. The separation membrane unit 1b is connected to an ATF pump 42, which is a membrane pump that operates pneumatically. Since the ATF pump 42 generates a reciprocating flow, the direction of flow can be changed in the flow channel 22. As a result, a culture solution 22 may flow into the separation membrane unit 1b and return to the culture vessel 10. A pump 40 is present in the middle of the flow channel 1c. The culture solution (the culture solution containing cells and metabolic decomposition products) is taken out from the inlet side of the flow channel 1 (the inlet side of the flow channel 1a) by the pump 40, and the taken-out culture solution 22 is injected into the purification unit from the outlet side of the flow channel 1 (the outlet side of the flow channel 1c) through the flow channel 1. Here, since the culture solution 24 injected into the purification unit from the outlet side of the flow channel 1 is filtered by the separation membrane 3, it does not contain the cells 12 (however, it contains metabolic decomposition products).

Further, in Fig. 1, a flow channel 2 (the inlet side of the flow channel 2) is connected to the outlet side of the purification unit 30. The culture solution in the purification unit 30 is taken out from the outlet side of the purification unit 30, and a taken-out culture solution 26 flows out from the outlet side of the flow channel 2 through the flow channel 2 and then is injected into the culture solution 20 of the culture vessel 10. Here, since the culture solution 26 taken out from the outlet side of the purification unit 30 has passed through the purification unit 30 filled with the above-described specific adsorbent, it is a purified culture solution from which metabolic decomposition products have been removed.

### [Metabolic decomposition product removal step]

In the metabolic decomposition product removal step, as described above, the culture solution 24 (the culture solution that does not contain cells) (however, containing metabolic decomposition products) filtered through the separation membrane 3 is allowed to pass through the purification unit 30, and metabolic decomposition products are removed from the culture solution 24 to obtain the culture solution 26 (the purified culture solution) as illustrated in Fig. 1. The culture solution 26 (the purified culture solution) is injected into the culture solution 20 of the culture vessel 10 as described above.

### [Culture step]

Further, in Fig. 1, in the culture step, cells are cultured in the culture solution 20 of the culture vessel 10. Here, as described above, the culture solution 26 (the purified culture solution) that has flown out of the flow channel 2 is injected into the culture solution of the culture vessel. That is, in the culture step, the cells 12 are cultured in the culture vessel 10 using the culture solution 26 (the purified culture solution).

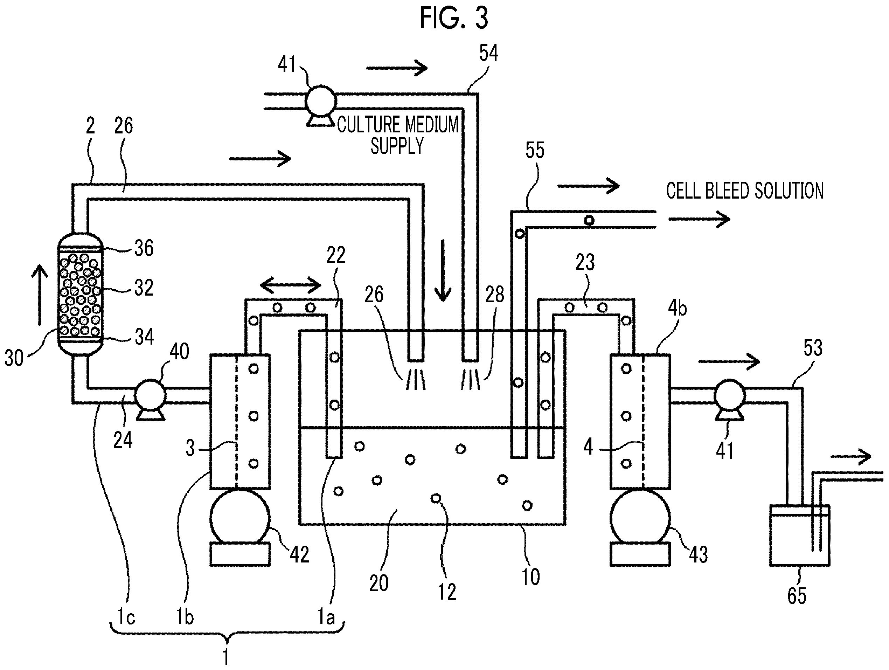

In the culture step, due to the reason that the effects of the present invention are more excellent, it is also a preferred aspect in which an unused culture solution is appropriately supplied to the culture vessel 10 in addition to the culture solution 26 (the perfusion culture) (Fig. 3). In this case, since the required amount of the unused culture solution for removing metabolic decomposition products can be reduced, it is possible to obtain the effect of reducing the amount of the culture medium to be used as compared with the normal perfusion culture.

Further, as a culture step, it is also possible to combine N-1 culture due to the reason that the effects of the present invention are more excellent. The N-1 culture is a culture in which the initial stage of cell proliferation is carried out by perfusion culture, and in a case where the number of cells is sufficiently increased, the cells are transferred to the fed batch. In a case of combining with the N-1 culture, it is possible to rapidly proliferate initial cells, and it is possible to suppress the decrease in the number of cells after the completion of perfusion as compared with the normal fed batch. The number of cells to be transferred from the initial perfusion culture to the fed batch is not particularly limited; however, it is preferably 8 M cells/mL to 130 M cells/mL, more preferably 10 M cells/mL to 100 M cells/mL, and most preferably 20 M cells/mL to 90 M cells/mL.

The timing of circulating the liquid in the column containing the specific adsorbent after the transfer to the fed batch is not particularly limited; however, it is preferably 10 M cells/ml or more, more preferably 15 M cells/ml or more, and still more preferably 20 M cells/ml or more.

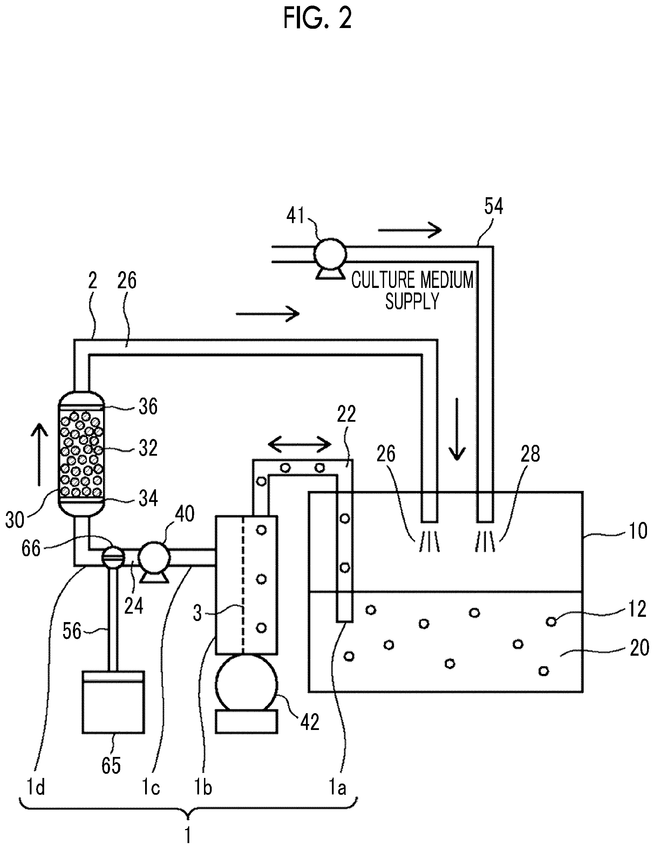

Fig. 2 and Fig. 3 are views schematically illustrating aspects of the preferred aspects of the method according to the embodiment of the present invention, where a treatment step with the specific adsorbent is combined with the perfusion culture or the N-1 culture.

Hereinafter, the production method for a product according to the present disclosure will be described in more detail with reference to the drawings.

Fig. 2 and Fig. 3 are views illustrating one example of the configuration of a cell culture device combined with N-1 culture and the perfusion culture, respectively, which is applicable to the implementation of the production method for a product according to the present disclosure, where one example of the membrane separation treatment by the tangential filtration method in a case where the hollow fiber-type separation membrane is used is schematically illustrated.

In Fig. 2, in addition to the device of Fig. 1, a flow channel 54 that supplies a fresh culture medium 28 with a pump 41 is connected to the culture vessel 10. Further, in the middle of the flow channel between the separation membrane unit 1b and the purification unit 30, there is a flow channel switching connector 66 capable of switching the flow channel, which is connected to a recovery tank 65 through a flow channel 56. At the initial stage of culture, the flow channel 1c is connected to the flow channel 56 by the switching of the flow channel switching connector 66, but it is not to the flow channel 1d. As a result, the fresh culture medium 28 is supplied to the culture vessel 10 through the flow channel 54, and the culture medium is recovered in the recovery tank 65 through the flow channel 56.

In Fig. 3, another separation membrane unit 4b is provided in addition to the device of Fig. 1. The separation membrane unit 1b is connected to the purification unit 30 and adsorbs metabolic decomposition products. On the other hand, the separation membrane unit 4b is continuously connected to the recovery tank 65, and the culture medium is recovered in the recovery tank 65 through the separation membrane unit 4b when the fresh culture medium 28 is sent from the flow channel 54 to the culture vessel 10.

None of the separation membrane units 1b and 4b allow cells to pass to the secondary side like the separation membrane unit 1b in Fig. 1.

Both the separation membrane units 1b and 4b may be an MF membrane or a UF membrane; however, the separation membrane unit 1b is preferably a UF membrane, and the separation membrane unit 4b is preferably an MF membrane.

It is preferable that a stirring device (not illustrated in the drawing) having a stirring blade is provided inside the culture container (the culture vessel) 10. In a case where the stirring blade is rotated, the culture medium accommodated in the culture container 10 is stirred, and thus homogeneity of the medium is maintained.

The cell culture device has a feed and a flow channel for supplying a nutritional component to the culture container 10 (not illustrated in the drawing).

The specific adsorbent may be or may not be exchanged during the culture period. In a case of carrying out exchange, it is preferable to carry out exchange while maintaining an aseptic state, and it is conceivable to install the specific adsorbent in the purification unit in advance or carry out exchange aseptically. From the viewpoint of ease of handling, it is preferable not to carry out the exchange. In a case where the exchange is not carried out, a sufficient amount of water-insoluble polymer may be installed in advance. In addition, for example, as a specific adsorbent to be used which has a buffering action, in a case where a specific adsorbent has a property of desorbing organic acids when a solution having a low organic acid concentration is added and has a property of adsorbing organic acids when the organic acid concentration increases, it is possible to maintain a state where a high cell proliferation rate is maintained for a long period of time. As a result, in a case of carrying out culture for a long period of 20 days or more, it is preferable to use a specific adsorbent having a buffering action.

Further, the purification unit may be always connected to the flow channel; however, the purification unit may be temporarily closed before and/or after the organic acid concentration is low or the feed is added. Here, in a case where the organic acid concentration is low, the purification unit is closed, for example, until the 2nd to 10th day and preferably 2nd to 5th day of the initial stage of cell proliferation, and then it is opened from the next day. In addition, "before and/or after the feed is added" means within 5 hours before the feed is added and within 24 hours after the feed is added. It is preferably within 2 hours before the feed is added and within 22 hours after the feed is added.

As described above, the present disclosure provides a production method for a product such as an antibody. The produced product can be used, for example, for a biopharmaceutical product, or regenerative medicine.

### [2] Antibody production method

The antibody production method according to the embodiment of the present invention is a method of producing an antibody by using the above-described method according to the embodiment of the present invention.

In the method according to the embodiment of the present invention described above, an antibody can be produced by using an antibody-producing cell as a cell.

### Examples

Hereinafter, the embodiment of the present disclosure will be more specifically described based on Examples, but the embodiment of the present disclosure is not limited to the following Examples.

### [1] Experiment using culture solution to which organic acid is forcibly added to culture solution as metabolic decomposition product of cell

### [Preparation of culture solution B containing organic acid]

23.2 mg (228 µmol) of isovaleric acid, 29.2 mg (331 µmol) of butyric acid, and 151.4 mg (788 µmol) of citric acid was added to a 1,000 mL volumetric flask, and the total volume was adjusted to 1,000 mL with a commercially available culture solution A (CD OptiCHO, manufactured by Thermo Fisher Scientific, Inc.) to prepare a culture solution B containing an organic acid.

### [Preparation of nutritional component C]

1.0 g of sodium pyruvate (manufactured by FUJIFILM Wako Pure Chemical Corporation), 10 mg of folic acid (manufactured by FUJIFILM Wako Pure Chemical Corporation), 10 mg of calcium pantothenate (manufactured by FUJIFILM Wako Pure Chemical Corporation), 10 mg of pyridoxine hydrochloride (manufactured by FUJIFILM Wako Pure Chemical Corporation), 10 mg of biotin (manufactured by FUJIFILM Wako Pure Chemical Corporation), and 5.0 g of disodium hydrogen phosphate dodecahydrate (manufactured by FUJIFILM Wako Pure Chemical Corporation) were added to a 1,000 mL volumetric flask, and the total volume was adjusted to 1,000 mL with ion exchange water to prepare a nutritional component.

### [Example 1]

The cell culture of Example 1 was carried out as follows.

### <Metabolic decomposition product removal step>

The culture solution B (20 mL) and a polymer having a quaternary ammonium base group (DIAION SA10A, manufactured by Mitsubishi Chemical Corporation, a trimethyl ammonium salt (type I), main chain: styrene-based, neutral salt decomposition capacity: 1.3 meq/ml, a water-insoluble polymer having a repeating unit represented by General Formula (PI) described above (here, in General Formula (PI), R₁, R₂, and R₃ represent a methyl group, and X represents Cl)) (corresponding to the above-described specific adsorbent) (1.96 g) (1.08 g in terms of the polymer content) were added to a sample bottle equipped with a 50 mL screw lid, and the resultant mixture was placed in a constant-temperature tank equipped with a shaker at 37°C and stirred for 2 hours. Here, the amount of the polymer having a quaternary ammonium base group with respect to the culture solution is 5% by mass.

Then, the treated culture solution was filtered through a 0.2 µm filter to remove the polymer. In this way, a purified culture solution E was obtained.

### <Culture step>

The purified culture solution E (9.5 mL) obtained in the metabolic decomposition product removal step was added to a 50 mL culture centrifuge tube, and 0.5 ml of an antibody-producing CHO cell solution of 4.0 × 10⁶ cells/mL was further added thereto, and then cells were seeded so that the number of cells was 2.0 × 10⁵ cells/mL and the total volume of the liquid was 10 mL. Antibody-producing CHO cells were prepared as described below.

Then, shaking culture was carried out at 180 rounds per minute (rpm) under an atmosphere of 37°C and 5% CO₂. The day on which seeding was carried out was set as the 0th day, and the cells were cultured for 4 days thereafter.

### (Antibody-producing CHO cell)

A vector containing a nucleic acid sequence encoding chimeric IgG1 (rituximab) was constructed, and the constructed vector was introduced into a CHO-DG44 cell (Thermo Fisher Scientific, Inc.), whereby a CHO-DG44 cell expressing IgG1 (an IgG1 cell) was prepared. The construction of the vector and the introduction thereof into the cell were carried out according to Example 2 of JP2016-517691A. This IgG1 cell was used for culture as the antibody-producing CHO cell.

### [Example 2]

The cell culture of Example 2 was carried out as follows.

### <Metabolic decomposition product removal step>

A metabolic decomposition product removal step was carried out according to the same procedure as in Example 1 to obtain a purified culture solution E.

### <Nutritional component addition step>

The nutritional component C (1.0 mL) was added to the obtained purified culture solution E to obtain a purified culture solution F to which the nutritional component C had been added.

### <Culture step>

The culture step was carried out according to the same procedure as in Example 1 except that the purified culture solution F was used instead of the purified culture solution E.

### [Reference Example]

The above-described culture solution A (9.5 mL) was added to a 50 mL culture centrifuge tube, and 0.5 ml of the above-described antibody-producing CHO cell solution of 4.0 × 10⁶ cells/mL was further added thereto, and then cells were seeded so that the number of cells was 2.0 × 10⁵ cells/mL and the total volume of the liquid was 10 mL.

Then, shaking culture was carried out at 180 rpm in an atmosphere of 37°C and 5% CO₂. The day on which seeding was carried out was set as the 0th day, and the cells were cultured for 4 days thereafter.

### [Comparative Example 1]

Cell culture was carried out according to the same procedure as in Example 1 except that in the culture step, the culture solution B was used instead of the purified culture solution E without carrying out the metabolic decomposition product removal step.

### [Comparative Example 2]

Cell culture was carried out according to the same procedure as in Example 2 except that in the nutritional component addition step, the culture solution B was used instead of the purified culture solution E without carrying out the metabolic decomposition product removal step. It is noted that culture solution B to which the nutritional component C has been added, which is obtained in the nutritional component addition step, is referred to as the culture solution G.

### [Evaluation]

Each of the solutions 4 days after culturing was evaluated as follows.

### <Cell proliferation property>

The number of cells was measured for each of the solutions 4 days after culturing using Vi-CELL (Beckman Coulter Inc.). The number of cells indicates the number of live cells. The same applies hereinafter.

Table 1 shows the number of seeded cells (the initial number of cells) and the number of cells 4 days after culturing (the number of cells after culturing). The evaluation result is shown in 5 stages from A to E, and regarding the number of cells after culturing, a case of 2.1 × 10⁶ or more is denoted as A, a case of less than 2.1 × 10⁶ and 1.9 × 10⁶ or more is denoted by B, a case of 1.9 × 10⁶ or less and 1.7 × 10⁶ or more is denoted as C, a case of less than 1.7 × 10⁶ and 1.6 × 10⁶ or more is denoted as D, and a case of less than 1.6 × 10⁶ is denoted as E. Practically, the evaluation stage is preferably A to D, more preferably A to C, still more preferably A to B, and particularly preferably A.

### <Antibody concentration>

For each of the solutions 4 days after culturing, the antibody concentration was measured using chromaster 5430 (Hitachi, Ltd.) HPLC and POROS 50A 4.6 × 50 mm (Applied Biosystems).

The results are shown in Table 1.

### <Organic acid content>

Regarding each of the used culture solutions (not including the antibody-producing CHO cell solution), the contents of the organic acids (isovaleric acid, butyric acid, and citric acid) were measured using the chromatographic separation using ICS-2100 and an Ion Pac AS11-HC column, manufactured by DIONEX Corporation.

The results are shown in Table 1. For example, the organic acid content (703 µM) of Example 1 is the organic acid content of the purified culture solution E.

**[Table 1]**

| | Culture solution A [mL] | Culture solution B [mL] | Culture solution (B + C) [mL] | Purified culture solution E [mL] | Purified culture solution F (E + C) [mL] | Antibody -producing CHO cell solution [mL] | Organic acid content] [µM] | Cell proliferation property | | | Antibody concentration [µg/mL] |
|---|---|---|---|---|---|---|---|---|---|---|---|
| | | | | | | | | Initial number of cells [10⁶ cells/mL] | Number of cells after culturing [10⁶ cells/mL] | Evaluation | |
| Reference Example | 9.5 | | | | | 0.5 | 0 | 0.2 | 2.64 | - | 79 |
| Comparative Example 1 | | 9.5 | | | | 0.5 | 1355 | 0.2 | 1.53 | E | 46 |
| Comparative Example 2 | | | 9.5 | | | 0.5 | 1290 | 0.2 | 1.5 | E | 44 |
| Example 1 | | | | 9.5 | | 0.5 | 703 | 0.2 | 1.75 | C | 55 |
| Example 2 | | | | | 9.5 | 0.5 | 674 | 0.2 | 2.55 | A | 77 |

In Table 1, the numerical values in the columns of "Culture solution" and "Antibody-producing CHO cell solution" indicate the amounts used in the culture step, where the unit is mL.

As can be seen from Table 1, Examples 1 and 2 in which the metabolic decomposition product removal step was carried out showed an excellent cell proliferation property as compared with Comparative Examples 1 and 2 in which the metabolic decomposition product removal step was not carried out. Among them, Example 2 including the nutritional component addition step showed a more excellent cell proliferation property.

### [2] Evaluation of specific adsorbent

The specific adsorbent was evaluated as follows.

### [Experiment A: Evaluation of organic acid adsorption performance of specific adsorbent]

The organic acid adsorption performance was evaluated for various specific adsorbents.

The above-described culture solution B (5 mL) and the specific adsorbent (0.27 g or 0.54 g in terms of the polymer content) shown in Table 2 below were added to a sample bottle equipped with a 50 ml screw lid, and the resultant mixture was placed in a constant-temperature tank equipped with a shaker at 37°C and stirred for 2 hours. Then, the treated culture solution was filtered through a 0.2 µm filter to remove the specific adsorbent. In this way, each of purified culture solutions (Experimental Examples A1 to A7) was prepared.

Regarding each of the obtained purified culture solutions, the amount of the organic acid was measured by using ion chromatography. Then, the residual rate of the organic acid after the treatment (the amount of the organic acid after the treatment/the amount of the organic acid before the treatment) was determined by setting the amount of the organic acid before the treatment to 100%.

The results are shown in Table 2. The evaluation result is shown in 5 stages from A to E, and regarding the residual rate of the organic acid, a case of less than 40% is denoted as, a case of 40% or more and less than 50% is denoted as B, a case of 50% or more and less than 70% is denoted as C, D, a case of 70% or more and less than 80% is denoted as D, and a case of 80% or more is denoted as E. Practically, A to C is preferable, A or B is more preferable, and A is particularly preferable.

**[Table 2]**

| | Specific adsorbent | | | | | Organic acid adsorption performance | |
|---|---|---|---|---|---|---|---|
| | No. | Product name | Functional group | Main chain | Using amount [g] | Residual rate of organic acid (%) | Evaluation |
| Experimental Example A1 | D-1 | SA10A | Trimethyl ammonium base group | Styrene-based | 0.27 | 34 | A |
| Experimental Example A2 | D-2 | SA11A | Trimethyl ammonium base group | Styrene-based | 0.27 | 34 | A |
| Experimental Example A3 | D-3 | SA20A | Dimethylhydroxyethyl ammonium base group | Styrene-based | 0.27 | 37 | A |
| Experimental Example A4 | D-4 | IRA458RF | Trimethyl ammonium base group | Acrylic | 0.27 | 37 | A |
| Experimental Example A5 | D-1 | SA10A | Trimethyl ammonium base group | Styrene-based | 0.54 | 33 | A |
| Experimental Example A6 | D-5 | PA316 | Trimethyl ammonium base group | Styrene-based | 0.27 | 37 | A |
| Experimental Example A7 | D-6 | WA30 | Dimethylamino group | Styrene-based | 0.27 | 60 | C |

In Table 2, the details of the specific adsorbent are as follows.
- D-1: DIAION SA10A, manufactured by Mitsubishi Chemical Corporation, a trimethyl ammonium salt (type I), main chain: styrene-based, neutral salt decomposition capacity: 1.3 meq/ml, a water-insoluble polymer having a repeating unit represented by General Formula (PI) described above (here, in General Formula (PI), R₁, R₂, and R₃ represent a methyl group, and X represents Cl)
- D-2: DIAION SA11A, manufactured by Mitsubishi Chemical Corporation, a trimethyl ammonium salt (type I), main chain: styrene-based, neutral salt decomposition capacity: 0.85 meq/ml, a water-insoluble polymer having a repeating unit represented by General Formula (PI) described above (here, in General Formula (PI), R₁, R₂, and R₃ represent a methyl group, and X represents Cl)
- D-3: DIAION SA20A, manufactured by Mitsubishi Chemical Corporation, a dimethylhydroxyethyl ammonium salt (type II), main chain: styrene-based, a water-insoluble polymer having a repeating unit represented by General Formula (PI) described above (here, in General Formula (PI), two of R₁, R₂, and R₃ represent a methyl group, one thereof represents a hydroxyethyl group, and X represents Cl)
- D-4: Amberlite IRA458RF, manufactured by ORGANO Corporation, a trimethyl ammonium salt (type I), main chain: acrylic, a water-insoluble polymer having a repeating unit represented by General Formula (P2) described above (here, in General Formula (P2), R₁, R₂, and R₃ represent a methyl group, R₄ represents a hydrogen atom, n represents 1 to 3, and X represents Cl)
- D-5: DIAION PA316, manufactured by Mitsubishi Chemical Corporation, a trimethyl ammonium salt (type I), main chain: styrene-based and porous type, a water-insoluble polymer having a repeating unit represented by General Formula (PI) described above (here, in General Formula (PI), R₁, R₂, and R₃ represent a methyl group, and X represents Cl)
- D-6: DIAION WA30, manufactured by Mitsubishi Chemical Corporation, dimethylamine, main chain: styrene type

From the comparison between Experimental Examples A1 to A7, it can be seen that the polymer having a quaternary ammonium base group has an excellent organic acid adsorption performance as compared with the polymer having an amino group.

Further, from the comparison between Experimental Example A1 and Experimental Example A5, it can be seen that the adsorbing amount is almost unchanged even in a case where the amount of the specific adsorbent used is doubled. As a result, it can be seen that this specific adsorbent has a buffering action.

Further, from the comparison between Experimental Example A1 and Experimental Example A6, it can be seen that the adsorbing amount is almost unchanged even in a case where a porous adsorbent having a wide adsorption site is used. From this fact as well, it can be seen that This specific adsorbent has a buffering action.

### [Experiment B: Evaluation of effect of specific adsorbent on culture solution]

In a case where the culture solution is brought into contact with the specific adsorbent, there is a possibility that the specific adsorbent adsorbs the nutritional components in the culture solution. The effect of various specific adsorbents on the culture solution was evaluated.

### <Reference Example B1>

The above-described culture solution A (9.5 mL) was added to a 50 mL culture centrifuge tube, and 0.5 ml of a CHO cell solution of 4.0 × 10⁶ cells/mL was further added thereto, and then cells were seeded so that the number of cells was 2.0 × 10⁵ cells/mL and the total volume of the liquid was 10 mL.

Then, shaking culture was carried out at 180 rpm in an atmosphere of 37°C and 5% CO₂. The day on which seeding was carried out was set as the 0th day, and the cells were cultured for 4 days thereafter.

Then, as described above, the cell proliferation property was evaluated. The results are shown in Table 3.

### <Experimental Examples B1 to B5>

Cell culture was carried out according to the same procedure as in Reference Example B1, and the cell proliferation property was evaluated in the same manner except that instead of the culture solution A, the culture solution A treated with (placed in a constant-temperature tank equipped with a shaker at 37°C and stirred for 2 hours) the specific adsorbent (0.27 g in terms of the polymer content) shown in Table 3 was used. The results are shown in Table 3.

**[Table 3]**

| | Specific adsorbent | | | Cell proliferation property | | |
|---|---|---|---|---|---|---|
| | Product name | Functional group | Main chain | Initial number of cells [10⁶ cells/mL] | Number of cells after culturing [10⁶ cells/mL] | Evaluation |
| Reference Example B 1 | - | - | - | 0.2 | 2.37 | - |
| Experimental Example B 1 | SA10A | Trimethyl ammonium base group | Styrene-based | 0.2 | 2.33 | A |
| Experimental Example B2 | SA11A | Trimethyl ammonium base group | Styrene-based | 0.2 | 2.30 | A |
| Experimental Example B3 | SA20A | Dimethylhydroxyethyl ammonium base group | Styrene-based | 0.2 | 2.20 | A |
| Experimental Example B4 | IRA458RF | Trimethyl ammonium base group | Acrylic | 0.2 | 2.00 | B |
| Experimental Example B5 | WA30 | Dimethylamino group | Styrene-based | 0.2 | 1.75 | C |

### [Conclusion]

Table 4 below summarizes Experimental Examples common to Experiment A (the evaluation of the organic acid adsorption performance) and Experiment B (the evaluation of the cell proliferation property).

**[Table 4]**

| Specific adsorbent | | | Organic acid adsorption performance | | Cell proliferation property | |
|---|---|---|---|---|---|---|
| Product name | Functional group | Main chain | Residual rate of organic acid (%) | Evaluation | Number of cells after culturing [10⁶ cells/mL] | Evaluation |
| SA10A | Trimethyl ammonium base group | Styrene-based | 34 | A | 2.33 | A |
| SA11A | Trimethyl ammonium base group | Styrene-based | 34 | A | 2.30 | A |
| SA20A | Dimethylhydroxyethyl ammonium base group | Styrene-based | 37 | A | 2.20 | A |
| IRA458RF | Trimethyl ammonium base group | Acrylic | 37 | A | 2.00 | B |
| WA30 | Dimethylamino group | Styrene-based | 60 | C | 1.75 | C |

From Table 4, it can be seen that the specific adsorbent of which the functional group is a quaternary ammonium base group exhibits a more excellent organic acid adsorption performance and cell proliferation property. Among the above, it can be seen that the specific adsorbent having a styrene-based main chain exhibits a further excellent cell proliferation property. Among the above, it can be seen that the specific adsorbent of which the functional group is a trimethyl ammonium base group exhibits a further excellent organic acid adsorption performance and cell proliferation property.

### [3] Continuous culture

Using the cell culture device illustrated in Fig. 1, cells were cultured and products (antibodies) were produced (Comparative Examples 1 to 3 and Examples 3 to 11). The above was carried out specifically as follows.

First, 600 mL of a culture medium (the culture solution A) (product name: CD OptiCHO, manufactured by Thermo Fisher Scientific, Inc., Inc.) was added to a culture container (a culture vessel) having a total volume of 2 L, the temperature thereof was held at 37°C, and aeration was carried out from the upper surface of the culture container with air at 1.8 mL/min and with CO₂ at 0.2 mL/min, and the culture medium was allowed to stand for one day. Next, an antibody-producing CHO cell solution was seeded so that the cell concentration (the number of cells) in the culture container was 5.0 × 10⁵ cells/mL and the liquid volume in the culture container was 0.8 L. The day on which seeding was carried out was set to the 0 days, and a feed (a fresh culture medium) (product name: Cell Boost 7a and 7b, a mixture manufactured by GE Healthcare) was daily supplied (a step of supplying a fresh culture medium) from the 3rd day after seeding, and after 4 days, the circulation of the culture solution to the adsorbent column (the purification unit) by a pump (the metabolic decomposition product removal step), filtration with the tangential filtration method (the separation treatment step), and the addition (daily addition of 0.08 L) of the nutritional component shown in Table 6 (the nutritional component addition step) were carried out. 250 g (125 g in terms of the polymer) of the specific adsorbent, of which the kind is shown in Table 6, was used. Details of the nutritional components are as shown in Table 5. The circulation to the adsorbent column (the purification unit) and the addition of the nutritional component shown in Table 6 were carried out until 20 days after the start of culture. The nutritional component was added at one time, and the circulation to the purification unit was carried at a rate of 0.4 L/hours (h). In the filtration by the tangential filtration method, the cell suspension was separated into a return solution having a cell concentration higher than that of the cell suspension and a permeated solution having a cell concentration lower than that of the cell suspension and containing an antibody as a product, and the return solution was returned to the culture container (step of returning the return solution to the culture solution). The feed was added daily, and the culture was continued for 21 days. Further, from 3 days after seeding, oxygen was supplied into the culture container at a flow rate of 1 mL/min from the lower surface side of the culture container using a single tube having an inner diameter of 2 mm, one end of which was inserted into the culture container.

It is noted that in Comparative Example 1, the metabolic decomposition product removal step was not carried out. Moreover, in Comparative Example 1 and Examples 3 to 4, the nutritional component addition step was not carried out.

### [Preparation of nutritional components C1 to C6]

The amounts shown in Table 5 (the amount shown is in terms of [mg]) were weighed and placed a 1,000 mL volumetric flask, an appropriate amount of ion exchange water was added thereto, and then the pH was prepared in a range of 0.55 to 0.7 with a 0.1 N sodium hydroxide aqueous solution. Then, the total volume was adjusted to 1,000 mL with ion exchange water to prepare nutritional components C1 to C6.

**[Table 5]**

| [mg] | C1 | C2 | C3 | C4 | C5 | C6 |
|---|---|---|---|---|---|---|
| Sodium pyruvate | 10 | | | | 1000 | 1000 |
| Calcium pantothenate | | | | | 10 | 10 |
| Folic acid | | 10 | | | 10 | 10 |
| Pyridoxine | | | | | 10 | 10 |
| Biotin | | | | | 10 | 10 |
| Disodium hydrogen phosphate dodecahydrate | | | 5000 | | 5000 | 5000 |
| Lactic acid | | | | | | 100 |
| Magnesium sulfate | | | | 10 | 10 | 10 |

### [Pump]

In the above test, a diaphragm-type reciprocating ATF pump (ATF2 (product name), manufactured by Repligen Corporation) was used as a cell suspension extraction filtration pump.

### [Measurement of antibody concentration]

A liquid high performance chromatograph (product name: Prominence, manufactured by Shimadzu Corporation) was used to quantify the antibody concentration. A sampling port was provided in the culture vessel (not illustrated in the drawing), and the sampled sample was filtrated and measured. As a column, Applied Biosystems POROS 50A having an inner diameter of 4.6 mm × 50 mm (manufactured by Thermo Fisher Scientific, Inc.) was used. Moreover, 20 mM phosphate buffer + 300 mM NaCl (pH of 7) and 20 mM phosphate buffer + 300 mM NaCl (pH of 2.8) were used as an eluant, and the measurement was carried out by a gradient elution method.

### [Cell]

The cell used in the above experiment was a CHO cell that produces an antibody.

### [Separation membrane]

As the separation membrane in the separation treatment step, a polyether sulfone (PES) filter having a pore diameter (Dp) of 0.2 µm manufactured by Repligen Corporation, which is a hollow fiber-type MF membrane, or a polyether sulfone (PES) filter manufactured by Repligen Corporation, which is a hollow fiber-type UF membrane was used. UF-A indicates a molecular weight cut-off of 10 kD, UF-B indicates a molecular weight cut-off of 1 kD, and UF-C indicates a molecular weight cut-off of 50 kD. Table 6 shows the separation membranes used in respective Examples and Comparative Examples.

### [Organic acid content]

Regarding the culture solution in the culture vessel on the 10th day of culture, the organic acid content was measured. The results are shown in Table 6. The method of measuring the organic content is as described above.

### [Peak number of cells]

The maximum value among the measured values of the live cell concentration during the duration of the culture days is shown in Table 6 as the peak number of cells. It can be said that the larger the peak number of cells, the better the cell proliferation property. In addition, the antibody concentration is described as a value 21 days after the start of culture.

**[Table 6]**

| | Culture solution A [mL] | Antibody -producing CHO cell solution [mL] | Specific adsorbent | Nutritional component | Separation membrane | Organic acid content after 10 days of culture [µM] | Cell proliferation property | | Relative ratio of antibody concentration [times] |
|---|---|---|---|---|---|---|---|---|---|
| | | | | | | | Initial number of cells [10⁶ cells/mL] | Peak umber of cells [10⁶ cells/mL] | |
| Comparative Example 1 | 600 | 200 | - | - | | 1921 | 0.5 | 29 | 1.0 |
| Example 3 | 600 | 200 | SA10A | - | MF | 735 | 0.5 | 36 | 1.1 |
| Example 4 | 600 | 200 | SA10A | - | UF-A | 740 | 0.5 | 45 | 1.2 |
| Example 5 | 600 | 200 | SA10A | C1 | UF-A | 743 | 0.5 | 48 | 1.3 |
| Example 6 | 600 | 200 | SA10A | C2 | UF-A | 728 | 0.5 | 52 | 1.4 |
| Example 7 | 600 | 200 | SA10A | C3 | UF-A | 736 | 0.5 | 50 | 1.3 |
| Example 8 | 600 | 200 | SA10A | C4 | UF-A | 748 | 0.5 | 52 | 1.4 |
| Example 9 | 600 | 200 | SA10A | C5 | UF-A | 743 | 0.5 | 66 | 1.6 |
| Example 10 | 600 | 200 | SA10A | C6 | UF-A | 748 | 0.5 | 69 | 1.7 |
| Example 11 | 600 | 200 | SA10A | C5 | UF-B | 742 | 0.5 | 67 | 1.7 |
| Example 12 | 600 | 200 | SA10A | C5 | UF-C | 744 | 0.5 | 44 | 1.2 |
| Example 13 | 600 | 200 | SA10A | C5 | MF | 743 | 0.5 | 40 | 1.1 |
| Example 14 | 600 | 200 | SA20A | C5 | UF-A | 821 | 0.5 | 63 | 1.5 |

In Comparative Example 1 and Example 11, the filterability was checked in a case where 800 mL of the liquid containing cells after culturing was filtered using a Millistak depth filter (surface area: 23 cm²). Five filtration filters were required in Comparative Example 1, whereas the entire amount could be filtered by using three filtration filters in Example 11. It can be seen that the culture solution obtained by the culture method using the present specific adsorbent is also excellent in filterability.

In Table 6, from the comparison between Comparative Example 1 and Example 3 and the comparison between Comparative Example 1 and Example 4, it can be seen that the mass of the inhibiting substances (the metabolic decomposition products) (the organic acids) is reduced by the metabolic decomposition product removal step, and the number of cells is increased.

In Table 6, from the comparison between Example 3 and Example 4 and the comparison between Example 9 and Example 13, it can be seen that better results have been obtained with the UF membrane than with the MF membrane as the separation membrane. It is conceived to be because the culturablity is improved by suppressing the contact of the small molecule component in the culture medium with the specific adsorbent. In general, as compared with Example 12 in which UF-C which is a UF membrane having a molecular weight cut-off of 50 kD and which is used for suppressing the antibody, better results were obtained with the UF membrane-A and the UF membrane-B, which are UF membranes having a molecular weight cut off of 10 kD and 1 kD, respectively. This suggests that suppression of components smaller than the antibody in the culture medium is particularly important.

In addition, good results were obtained in a case where the nutritional component was added as compared with a case where it was not added, and Examples 5 to 10 to which C1 to C6 were respectively added were superior to Example 4 in which no nutritional component was added. Since the addition of nutrients is effective, it is conceived that it is particularly effective to monitor the culture state and carry out culture while controlling the nutrients to be added daily.

### [4] Combination with N-1 culture

Using the cell culture device illustrated in Fig. 2, cells were cultured and products (antibodies) were produced (Comparative Examples 4 and 5 and Examples 15 and 16). The above was carried out specifically as follows.

First, a culture container having a total volume of 2 L was used as the culture container (the culture vessel) 10. 600 mL of the culture medium (the culture solution A) (product name: CD OptiCHO, manufactured by Thermo Fisher Scientific, Inc., Inc.) was added thereto, the temperature thereof was held at 37°C, and aeration was carried out from the upper surface of the culture container with air at 1.8 mL/min and with CO₂ at 0.2 mL/min, and the culture medium was allowed to stand for one day. Next, an antibody-producing CHO cell solution was seeded so that the cell concentration (the number of cells) in the culture container was 5.0 × 10⁵ cells/mL and the liquid volume in the culture container was 0.8 L. During the culture period, air having a flow rate of 0.1 L/min was supplied from the upper surface in the culture container 10.

From 3 days after seeding, filtration was carried out by the tangential flow filtration method, and the culture medium was discarded in the recovery tank 65 by the flow channel switching connector 66. A hollow fiber membrane filter (molecular weight cut-off: 10 kD) of a UF membrane manufactured by Repligen Corporation was used for filtration. For replenishing the amount of the liquid lost from the culture system by filtration, the supply (the supplying of a fresh culture medium into the culture container) of a fresh culture medium (a mixture of a culture medium represented by a product name of CD OptiCHO, manufactured by Thermo Fisher Scientific, Inc. and a culture medium represented by a product name of Cell Boost 7a and 7b, manufactured by GE Healthcare) from the flow channel 54 was carried out, and culture was carried out. The above filtration (the discarding of the culture medium to the recovery tank 65) and the supply of the fresh culture medium were carried out at 1.0 L/day. In the filtration by the tangential flow filtration method, the cell suspension was separated into a return solution having a cell concentration higher than that of the cell suspension and a permeated solution having a cell concentration lower than that of the cell suspension, and the return solution was returned to the inside of the culture container (the returning of the return solution to the culture container). Further, from 3 days after seeding, oxygen was supplied into the culture container at a flow rate of 1 mL/min from the lower surface side of the culture container using a single tube having an inner diameter of 2 mm, one end of which was inserted into the culture container.

In the above culture, from the 9th day on which the cell concentration reached 70 × 10⁶ cells/mL, the supply of the culture medium from the flow channel 54 and the discarding of the culture medium to the recovery tank 65 were stopped. The flow channel switching connector 66 was switched, and the circulation of the culture solution to the purification unit 30 by the pump (the metabolic decomposition product removal step) was carried out as it was at 0.4 L/h, and the filtration by the tangential filtration method (the separation treatment step) was carried out. 250 g (125 g in terms of the polymer) of the specific adsorbent, of which the kind is shown in Table 7, was used. In the filtration by the tangential filtration method, the cell suspension was separated into a return solution having a cell concentration higher than that of the cell suspension and a permeated solution having a cell concentration lower than that of the cell suspension and containing an antibody as a product, and the return solution was returned to the culture container (step of returning the return solution to the culture solution).

During the circulation to the purification unit 30, 0.08 L of the nutritional component shown in Table 7 was added daily (the nutritional component addition step). Details of the nutritional components are as shown in Table 5. The circulation to the purification unit was carried out until 20 days after the start of culture. In this state, the culture was continued for 21 days.

It is noted that in Comparative Examples 4 to 5, the metabolic decomposition product removal step and the separation treatment step were not carried out. Moreover, in Comparative Example 4 and Example 15, the nutritional component addition step was not carried out.

### [Measurement of antibody concentration]

A liquid high performance chromatograph (product name: Prominence, manufactured by Shimadzu Corporation) was used to quantify the antibody concentration. As a column, Applied Biosystems POROS 50A having an inner diameter of 4.6 mm × 50 mm (manufactured by Thermo Fisher Scientific, Inc.) was used. Moreover, 20 mM phosphate buffer + 300 mM NaCl (pH of 7) and 20 mM phosphate buffer + 300 mM NaCl (pH of 2.8) were used as an eluant, and the measurement was carried out by a gradient elution method.

### [Number of live cells on 21st day]

The number of live cells on the 21st day is shown in Table 7. It can be said that the larger the number of live cells on the 21st day, the better the cell proliferation property.

**[Table 7]**

| | Culture solution A [mL] | Antibody -producing CHO cell solution [mL] | Specific adsorbent | Nutritional component | Separation membrane | Cell proliferation property | | | Relative ratio of antibody concentration [times] |
|---|---|---|---|---|---|---|---|---|---|
| | | | | | | Initial number of cells [10⁶ cells/mL] | Number of live cells at 9th day [10⁶ cells/mL] | Number of live cells at 21st day [10⁶ cells/mL] | |
| Comparative Example 4 | 600 | 200 | - | - | - | 0.5 | 70 | 17 | 1 |
| Comparative Example 5 | 600 | 200 | - | C5 | - | 0.5 | 70 | 17 | 1 |
| Example 15 | 600 | 200 | SA10A | - | UF | 0.5 | 70 | 58 | 1.4 |
| Example 16 | 600 | 200 | SA10A | C5 | UF | 0.5 | 70 | 64 | 1.5 |

In Table 7, the number of live cells on the 21st day is significantly reduced in Comparative Example 4 and Comparative Example 5; however, the decrease in the number of live cells is small in Examples 15 and 16 in which the metabolic decomposition product removal step is carried out, the number of live cells can be maintained high during the culture period (excellent in cell proliferation property), and the antibody concentration also increases.

### [5] Combination with perfusion culture

Cell culture was carried out by the perfusion culture method using the cell culture device having the configuration illustrated in Fig. 3 (Comparative Examples 6 and 7, and Examples 17 and 18). The above was carried out specifically as follows.

First, 600 mL of the culture medium (the culture solution A) (product name: CD OptiCHO, manufactured by Thermo Fisher Scientific, Inc.) was added to a culture container having a total volume of 2 L manufactured by ABLE INC., as the culture container (the culture vessel) 10, the temperature thereof was held at 37°C, and aeration was carried out from the upper surface of the culture container with air at 1.8 mL/min and with CO₂ at 0.2 mL/min, and the medium was allowed to stand for 1 day. Next, an antibody-producing CHO cell solution was seeded so that the cell concentration (the number of cells) in the culture container was 5.0 × 10⁵ cells/mL and the liquid volume in the culture container was 0.8 L. The supply of fresh culture medium and the extraction of the cell suspension from the culture container 10 (the discarding of the culture medium in the recovery tank 65) were carried out after the third day from the start of the culture. From 3 days after seeding, filtration was carried out by the tangential flow filtration method, and the culture medium was discarded in the recovery tank 65. A hollow fiber membrane filter (MF) manufactured by Repligen Corporation was used for the separation membrane 4 of the separation membrane unit 4b. For replenishing the amount of the liquid lost from the culture system by filtration, the supply (the supplying of a fresh culture medium in the culture container) of a fresh culture medium (a mixture of a culture medium represented by a product name of CD OptiCHO, manufactured by Thermo Fisher Scientific, Inc. and a medium represented by a product name of Cell Boost 7a and 7b, manufactured by GE Healthcare) from the flow channel 54 was carried out. The above filtration (the discarding of the culture medium to the recovery tank 65) and the supply of the fresh culture medium were carried out at the rate described in the column of "Fresh culture medium supply rate" of Table 8.

Further, from the 4th day after the start of the culture, the filtration (the separation membrane 3) by the tangential filtration method (the separation treatment step) and the circulation of the culture solution to the purification unit 30 (the metabolic decomposition product removal step) were carried out at a rate of 0.4 L/h. A hollow fiber membrane filter (molecular weight cut-off: 10 kD) of a UF membrane manufactured by Repligen Corporation was used for filtration. 250 g (125 g in terms of the polymer) of the specific adsorbent, of which the kind is shown in Table 8, was used. The circulation to the purification unit 30 was carried out until 20 days after the start of culture. In addition, 0.08 L of the nutritional component shown in Table 8 was added daily during the circulation (the nutritional component addition step). Details of the nutritional components are as shown in Table 5.

In the filtration by the tangential flow filtration method, the cell suspension was separated into a return solution having a cell concentration higher than that of the cell suspension and a permeated solution having a cell concentration lower than that of the cell suspension, and the return solution was returned to the inside of the culture container (the returning of the return solution to the culture container). From the same day (from 3 days after seeding), oxygen was supplied into the culture container at a flow rate of 1 mL/min from the lower surface side of the culture container using a single tube having an inner diameter of 2 mm, one end of which was inserted into the culture container.

In the above culture, from the day in a case where the cell concentration reached 110 × 10⁶ cells/mL, an operation of extracting a predetermined cell suspension volume from the culture container through the flow channel 55 (a cell bleeding operation) was carried out once a day so that the average cell concentration (the average value of the cell concentration at the two time points by a twice-daily measurement of the cell concentration) was about 110 × 10⁶ cells/mL, and the culture was continued to the 21st day after seeding.

It is noted that in Comparative Examples 6 and 7, the metabolic decomposition product removal step was not carried out. Moreover, in Comparative Example 6 and Example 17, the nutritional component addition step was not carried out.

**[Table 8]**

| | Culture solution A [mL] | Antibody -producing CHO cell solution [mL] | Specific adsorbent | Fresh culture medium supply rate [L/day] | Nutritional component | Separation membrane 4 (recovery tank side) | Separation membrane 3 (purification unit side) | Cell proliferation property | | Relative ratio of average antibody concentration [times] | Total using amount of culture medium[L] | Relative ratio of total antibody amount per batch [times] |
|---|---|---|---|---|---|---|---|---|---|---|---|---|
| | | | | | | | | Initial number of cells [10⁶ cells/mL ] | Number of set cells [10⁶ cells/mL] | | | |
| Comparative Example 6 | 600 | 200 | - | 1.00 | - | MF | | 0.5 | 110 | 1.0 | 18.8 | 1.0 |
| Comparative Example 7 | 600 | 200 | - | 1.00 | C5 | MF | | 0.5 | 110 | 0.9 | 20.2 | 1.0 |
| Example 17 | 600 | 200 | SA10A | 0.50 | - | MF | UF | 0.5 | 110 | 2.0 | 9.8 | 1.0 |
| Example 18 | 600 | 200 | SA10A | 0.50 | C5 | MF | UF | 0.5 | 110 | 2.1 | 11.2 | 1.2 |

In a case where the number of maintenance cells (the number of set cells) is set to the same number in the combination with perfusion culture, as compared with the total using amount in the typical perfusion culture shown in Comparative Example 6 and Comparative Example 7, it is possible to reduce the total using amount of the culture medium required for obtaining the same amount or more of the antibody to about half in Examples 17 and 18 in which the metabolic decomposition product removal step has been carried out. From this, as compared with Comparative Examples 6 and 7, the used culture medium can be reduced in Examples 17 and 18, which is excellent in terms of cost and environment.

The amount of the fresh culture medium per day with respect to 0.8 L of the culture vessel liquid volume, which is required to maintain 110 × 10⁶ cells/mL, is 1.0 L in Comparative Examples 6 and 7, whereas it is 0.5 L or less in Examples 17 and 18, which is better. That is, although the amount of the culture medium required to maintain 110 × 10⁶ cells/mL is typically 1.1 vvd, it can be 0.63 vvd or less. Here, vvd is a unit that indicates an amount obtained by dividing the culture medium liquid amount per day by the total amount of the culture vessel liquid.

### Explanation of References

1: flow channel 1
1a: flow channel
1b, 4b: separation membrane unit
1c, 1d: flow channel
2: flow channel 2
3, 4: separation membrane
10: culture vessel
12: cell
20: culture solution
22, 23: culture solution
24: culture solution
26: culture solution (purified culture solution)
28: fresh culture medium
30: purification unit
32: specific adsorbent
34: filter
36: filter
40,41: pump
42, 43: ATF pump
53, 54, 55, 56: flow channel
65: recovery tank
66: flow channel switching connector

## Claims

1. A cell culture method comprising:
a metabolic decomposition product removal step of bringing a culture solution containing a metabolic decomposition product into contact with a polymer having a quaternary ammonium base group or an amino group and removing the metabolic decomposition product from the culture solution to a purified culture solution; and
a cell culture step of culturing cells using the purified culture solution.

2. The cell culture method according to claim 1,
wherein the culture solution containing the metabolic decomposition product is a culture solution taken out from the culture solution in the culture step.

3. The cell culture method according to claim 1 or 2,
wherein the culture solution containing the metabolic decomposition product does not contain cells.

4. The cell culture method according to claim 3,
wherein the culture solution containing the metabolic decomposition product is a culture solution obtained by filtering a culture solution taken out from the culture solution in the culture step.

5. The cell culture method according to claim 4,
wherein the metabolic decomposition product removal step is a step of passing the filtered culture solution through a purification unit filled with the polymer and removing the metabolic decomposition product from the culture solution to obtain the purified culture solution,
the culture step is a step of culturing cells using the purified culture solution in a culture vessel,
an inlet side of the purification unit and the culture vessel are connected by a flow channel having a separation membrane in the middle of the flow channel, and
the filtered culture solution is a culture solution obtained by filtering, with the separation membrane, the culture solution taken out from the culture solution in the culture step through the flow channel.

6. The cell culture method according to any one of claims 1 to 5, further comprising a nutritional component addition step of adding a nutritional component to the purified culture solution obtained in the metabolic decomposition product removal step to obtain a purified culture solution to which the nutritional component has been added.

7. The cell culture method according to claim 6,
wherein the nutritional component contains at least one selected from the group consisting of amino acids, vitamins, energy sources, osmotic pressure regulators, pH buffers, trace metal elements, surfactants, and growth supporting factors.

8. The cell culture method according to claim 6 or 7,
wherein the nutritional component contains at least one selected from the group consisting of vitamins, energy sources, pH buffers, and trace metal elements.

9. The cell culture method according to any one of claims 1 to 8,
wherein the metabolic decomposition product is an organic acid.

10. The cell culture method according to claim 9,
wherein the organic acid includes at least any one of isovaleric acid, butyric acid, or citric acid.

11. The cell culture method according to any one of claims 1 to 10,
wherein the polymer is a water-insoluble polymer having a quaternary ammonium base group.

12. The cell culture method according to claim 11,
wherein the water-insoluble polymer has a partial structure represented by General Formula (p1), 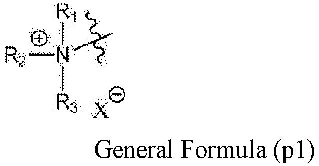
in General Formula (p1), R₁, R₂, and R₃ each independently represent a hydrogen atom, an alkyl group having 1 to 12 carbon atoms, or a phenyl group, and may have a hydroxyl group as a substituent, and X represents OH, Cl, Br, OTs, or PF₆, where Ts represents CH₃-C₆H₄-SO₂-.

13. The cell culture method according to claim 12,
wherein in General Formula (p1), R₁, R₂, and R₃ are each independently a methyl group or a hydroxyethyl group, and X is Cl, Br, OTs, or PF₆.

14. The cell culture method according to claim 11,
wherein the water-insoluble polymer has a repeating unit represented by General Formula (PI) or (P2), 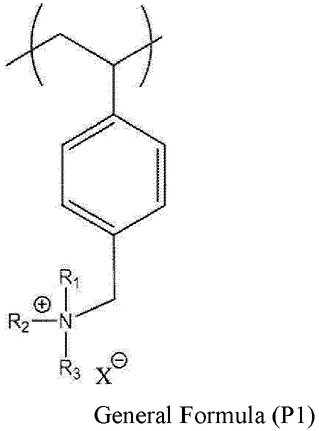 in General Formula (PI), R₁, R₂, and R₃ each independently represent a hydrogen atom, an alkyl group having 1 to 12 carbon atoms, or a phenyl group, and X represents OH, Cl, Br, OTs, or PF₆, where Ts represents CH₃-C₆H₄-SO₂-, 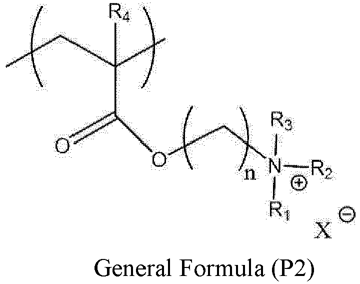 in General Formula (P2), R₁, R₂, R₃, and R₄ each independently represent a hydrogen atom, an alkyl group having 1 to 12 carbon atoms, or a phenyl group, n represents an integer of 1 to 8, and X represents OH, Cl, Br, OTs, or PF₆, where Ts represents CH₃-C₆H₄-SO₂-.

15. The cell culture method according to claim 14,
wherein the water-insoluble polymer has a repeating unit represented by General Formula (PI).

16. The cell culture method according to claim 15,
wherein in General Formula (PI), R₁, R₂, and R₃ are a methyl group, and X is Cl or Br.

17. The cell culture method according to any one of claims 1 to 16,
wherein the cell is a floating cell.

18. The cell culture method according to any one of claims 1 to 17,
wherein the cell is an animal cell.

19. The cell culture method according to any one of claims 1 to 18,
wherein the cell is a CHO cell.

20. The cell culture method according to any one of claims 1 to 19,
wherein the cell is a productive cell.

21. The cell culture method according to claim 20,
wherein the productive cell is an antibody-producing cell.

22. An antibody production method using the cell culture method according to claim 21.

23. An organic acid removal method, comprising removing at least one organic acid selected from the group consisting of isovaleric acid, butyric acid, and citric acid from a culture solution by using the cell culture method according to claim 19.

24. An antibody produced by using the cell culture method according to claim 21.
